# EUROPEAN PATENT SPECIFICATION

(11) **EP 2 822 573 B1**
(45) Date of publication and mention of the grant of the patent: **16.08.2017**
(21) Application number: 13709521.2
(22) Date of filing: 08.03.2013
(51) Int. Cl.: A61K 38/17, A61P 35/00

(54) **TREATMENT OF CANCER**
KREBSBEHANDLUNG
TRAITEMENT CONTRE LE CANCER

(30) Priority: 09.03.2012 US 201261608872 P
(43) Date of publication of application: 14.01.2015
(73) Proprietor: UCL Business Plc., London W1T 4TP (GB)
(72) Inventor: GREENWOOD, John, London EC1V 9EL (GB); MOSS, Stephen, London EC1V 9EL (GB); WANG, Xiaomeng, London EC1V 9EL (GB)
(74) Representative: J A Kemp
(86) International application number: PCT/GB2013/050580
(87) International publication number: WO 2013/132267

(56) References cited:
- WO-A1-2011/027129
- LYNCH J; FAY J; MEEHAN M; BRYAN K; WATTERS K; MURPHY D; STALLINGS R: "MiRNA-335 suppresses neuroblastoma cell invasiveness by direct targeting of multiple genes from the non-canonical TGF[ signalling pathway", CARCINOGENESIS, 1 March 2012 (2012-03-01), XP002696201, cited in the application
- ANDERSEN JOHN D ET AL: "Leucine-rich alpha-2-glycoprotein-1 is upregulated in sera and tumors of ovarian cancer patients", JOURNAL OF OVARIAN RESEARCH, BIOMED CENTRAL LTD, LONDON, UK, vol. 3, no. 1, 10 September 2010 (2010-09-10), page 21, XP021084161, ISSN: 1757-2215, DOI: 10.1186/1757-2215-3-21
- LADD J J ET AL: "Increased plasma levels of the APC-interacting protein MAPRE1, LRG1, and IGFBP2 preceding a diagnosis of colorectal cancer in women", CANCER PREVENTION RESEARCH 2012 AMERICAN ASSOCIATION FOR CANCER RESEARCH INC. USA, vol. 5, no. 4, 1 January 2012 (2012-01-01) , pages 655-664, XP009169190, ISSN: 1940-6207

### SEQUENCE LISTING

<110> UCL Business PLC
<120> Treatment of cancer
<130> N.116113B
<150> US 61/608,872
   <151> 2012-03-09
<160> 8
<170> PatentIn version 3.5
<210> 1
   <211> 1780
   <212> DNA
   <213> Homo sapiens
<400> 1
   gcagagctac catgtcctct tggagcagac agcgaccaaa aagcccaggg ggcattcaac 60
   cccatgtttc tagaactctg ttcctgctgc tgctgttggc agcctcagcc tggggggtca 120
   ccctgagccc caaagactgc caggtgttcc gctcagacca tggcagctcc atctcctgtc 180
   aaccacctgc cgaaatcccc ggctacctgc cagccgacac cgtgcacctg gccgtggaat 240
   tcttcaacct gacccacctg ccagccaacc tcctccaggg cgcctctaag ctccaagaat 300
   tgcacctctc cagcaatggg ctggaaagcc tctcgcccga attcctgcgg ccagtgccgc 360
   agctgagggt gctggatcta acccgaaacg ccctgaccgg gctgcccccg ggcctcttcc 420
   aggcctcagc caccctggac accctggtat tgaaagaaaa ccagctggag gtcctggagg 480
   tctcgtggct acacggcctg aaagctctgg ggcatctgga cctgtctggg aaccgcctcc 540
   ggaaactgcc ccccgggctg ctggccaact tcaccctcct gcgcaccctt gaccttgggg 600
   agaaccagtt ggagaccttg ccacctgacc tcctgagggg tccgctgcaa ttagaacggc 660
   tacatctaga aggcaacaaa ttgcaagtac tgggaaaaga tctcctcttg ccgcagccgg 720
   acctgcgcta cctcttcctg aacggcaaca agctggccag ggtggcagcc ggtgccttcc 780
   agggcctgcg gcagctggac atgctggacc tctccaataa ctcactggcc agcgtgcccg 840
   aggggctctg ggcatcccta gggcagccaa actgggacat gcgggatggc ttcgacatct 900
   ccggcaaccc ctggatctgt gaccagaacc tgagcgacct ctatcgttgg cttcaggccc 960
   aaaaagacaa gatgttttcc cagaatgaca cgcgctgtgc tgggcctgaa gccgtgaagg 1020
   gccagacgct cctggcagtg gccaagtccc agtgagacca ggggcttggg ttgagggtgg 1080
   ggggtctggt agaacactgc aacccgctta acaaataatc ctgcctttgg ccgggtgcgg 1140
   gggctcacgc ctgtaatccc agcactttgg gaggcccagg tgggcggatc acgaggtcag 1200
   gagatcgaga ccatcttggc taacatggtg aaaccctgtc tctactaaaa atataaaaaa 1260
   ttagccaggc gtggtggtgg gcacctgtag tcccagcaac tcgggaggct gaggcaggag 1320
   aatggcgtga acttgggagg cggagcttgc ggtgagccaa gatcgtgcca ctgcactcta 1380
   gcctgggcga cagagcaaga ctgtctcaaa aaaattaaaa ttaaaattaa aaacaaataa 1440
   tcctgccttt tacaggtgaa actcggggct gtccatagcg gctgggaccc cgtttcatcc 1500
   atccatgctt cctagaacac acgatgggct ttccttaccc atgcccaagg tgtgccctcc 1560
   gtctggaatg ccgttccctg tttcccagat ctcttgaact ctgggttctc ccagcccctt 1620
   gtccttcctt ccagctgagc cctggccaca ctggggctgc ctttctctga ctctgtcttc 1680
   cccaagtcag ggggctctct gagtgcaggg tctgatgctg agtcccactt agcttggggt 1740
   cagaaccaag gggtttaata aataaccctt gaaaactgga 1780
<210> 2
   <211> 347
   <212> PRT
   <213> Homo sapiens
<400> 2
   Met Ser Ser Trp Ser Arg Gln Arg Pro Lys Ser Pro Gly Gly Ile Gln
   1               5                   10                  15
   Pro His Val Ser Arg Thr Leu Phe Leu Leu Leu Leu Leu Ala Ala Ser
               20                  25                  30
   Ala Trp Gly Val Thr Leu Ser Pro Lys Asp Cys Gln Val Phe Arg Ser
           35                  40                  45
   Asp His Gly Ser Ser Ile Ser Cys Gln Pro Pro Ala Glu Ile Pro Gly
       50                  55                  60
   Tyr Leu Pro Ala Asp Thr Val His Leu Ala Val Glu Phe Phe Asn Leu
   65                  70                  75                  80
   Thr His Leu Pro Ala Asn Leu Leu Gln Gly Ala Ser Lys Leu Gln Glu
                   85                  90                  95
   Leu His Leu Ser Ser Asn Gly Leu Glu Ser Leu Ser Pro Glu Phe Leu
               100                 105                 110
   Arg Pro Val Pro Gln Leu Arg Val Leu Asp Leu Thr Arg Asn Ala Leu
           115                 120                 125
   Thr Gly Leu Pro Pro Gly Leu Phe Gln Ala Ser Ala Thr Leu Asp Thr
       130                 135                 140
   Leu Val Leu Lys Glu Asn Gln Leu Glu Val Leu Glu Val Ser Trp Leu
   145                 150                 155                 160
   His Gly Leu Lys Ala Leu Gly His Leu Asp Leu Ser Gly Asn Arg Leu
                   165                 170                 175
   Arg Lys Leu Pro Pro Gly Leu Leu Ala Asn Phe Thr Leu Leu Arg Thr
               180                 185                 190
   Leu Asp Leu Gly Glu Asn Gln Leu Glu Thr Leu Pro Pro Asp Leu Leu
           195                 200                 205
   Arg Gly Pro Leu Gln Leu Glu Arg Leu His Leu Glu Gly Asn Lys Leu
       210                 215                 220
   Gln Val Leu Gly Lys Asp Leu Leu Leu Pro Gln Pro Asp Leu Arg Tyr
   225                 230                 235                 240
   Leu Phe Leu Asn Gly Asn Lys Leu Ala Arg Val Ala Ala Gly Ala Phe
                   245                 250                 255
   Gln Gly Leu Arg Gln Leu Asp Met Leu Asp Leu Ser Asn Asn Ser Leu
               260                 265                 270
   Ala Ser Val Pro Glu Gly Leu Trp Ala Ser Leu Gly Gln Pro Asn Trp
           275                 280                 285
   Asp Met Arg Asp Gly Phe Asp Ile Ser Gly Asn Pro Trp Ile Cys Asp
       290                 295                 300
   Gln Asn Leu Ser Asp Leu Tyr Arg Trp Leu Gln Ala Gln Lys Asp Lys
   305                 310                 315                 320
   Met Phe Ser Gln Asn Asp Thr Arg Cys Ala Gly Pro Glu Ala Val Lys
                   325                 330                 335
   Gly Gln Thr Leu Leu Ala Val Ala Lys Ser Gln
               340                 345
<210> 3
   <211> 24
   <212> PRT
   <213> Homo sapiens
<400> 3
   Leu Gln Glu Leu His Leu Ser Ser Asn Gly Leu Glu Ser Leu Ser Pro
   1               5                   10                  15
   Glu Phe Leu Arg Pro Val Pro Gln
                                   20
<210> 4
   <211> 24
   <212> PRT
   <213> Homo sapiens
<400> 4
   Leu Asp Met Leu Asp Leu Ser Asn Asn Ser Leu Ala Ser Val Pro Glu
   1               5                   10                  15
   Gly Leu Trp Ala Ser Leu Gly Gln
               20
<210> 5
   <211> 26
   <212> PRT
   <213> Homo sapiens
<400> 5
   Lys Met Phe Ser Gln Asn Asp Thr Arg Cys Ala Gly Pro Glu Ala Val
   1               5                   10                  15
   Lys Gly Gln Thr Leu Leu Ala Val Ala Lys
               20                  25
<210> 6
   <211> 342
   <212> PRT
   <213> Mus musculus
<400> 6
   Met Val Ser Trp Gln His Gln Gly Ser Leu Gln Asp Leu Lys Thr Cys
   1               5                   10                  15
   Leu Ala Arg Thr Leu Phe Leu Leu Ala Leu Leu Gly Arg Val Ser Ser
               20                  25                  30
   Leu Lys Glu Cys Leu Ile Leu Gln Ser Ala Glu Gly Ser Thr Val Ser
           35                  40                  45
   Cys His Gly Pro Thr Glu Phe Pro Ser Ser Leu Pro Ala Asp Thr Val
       50                  55                  60
   His Leu Ser Val Glu Phe Ser Asn Leu Thr Gln Leu Pro Ala Ala Ala
   65                  70                  75                  80
   Leu Gln Gly Cys Pro Gly Leu Arg Glu Leu His Leu Ser Ser Asn Arg
                   85                  90                  95
   Leu Gln Ala Leu Ser Pro Glu Leu Leu Ala Pro Val Pro Arg Leu Arg
               100                 105                 110
   Ala Leu Asp Leu Thr Arg Asn Ala Leu Arg Ser Leu Pro Pro Gly Leu
           115                 120                 125
   Phe Ser Thr Ser Ala Asn Leu Ser Thr Leu Val Leu Arg Glu Asn Gln
       130                 135                 140
   Leu Arg Glu Val Ser Ala Gln Trp Leu Gln Gly Leu Asp Ala Leu Gly
   145                 150                 155                 160
   His Leu Asp Leu Ala Glu Asn Gln Leu Ser Ser Leu Pro Ser Gly Leu
                   165                 170                 175
   Leu Ala Ser Leu Gly Ala Leu His Thr Leu Asp Leu Gly Tyr Asn Leu
               180                 185                 190
   Leu Glu Ser Leu Pro Glu Gly Leu Leu Arg Gly Pro Arg Arg Leu Gln
           195                 200                 205
   Arg Leu His Leu Glu Gly Asn Arg Leu Gln Arg Leu Glu Asp Ser Leu
       210                 215                 220
   Leu Ala Pro Gln Pro Phe Leu Arg Val Leu Phe Leu Asn Asp Asn Gln
   225                 230                 235                 240
   Leu Val Gly Val Ala Thr Gly Ser Phe Gln Gly Leu Gln His Leu Asp
                   245                 250                 255
   Met Leu Asp Leu Ser Asn Asn Ser Leu Ser Ser Thr Pro Pro Gly Leu
               260                 265                 270
   Trp Ala Phe Leu Gly Arg Pro Thr Arg Asp Met Gln Asp Gly Phe Asp
           275                 280                 285
   Ile Ser His Asn Pro Trp Ile Cys Asp Lys Asn Leu Ala Asp Leu Cys
       290                 295                 300
   Arg Trp Leu Val Ala Asn Arg Asn Lys Met Phe Ser Gln Asn Asp Thr
   305                 310                 315                 320
   Arg Cys Ala Gly Pro Glu Ala Met Lys Gly Gln Arg Leu Leu Asp Val
                   325                 330                 335
   Ala Glu Leu Gly Ser Leu
               340
<210> 7
   <211> 248
   <212> PRT
   <213> Mus musculus
<400> 7
   His Leu Ser Ser Asn Arg Leu Gln Ala Leu Ser Pro Glu Leu Leu Ala
   1               5                   10                  15
   Pro Val Pro Arg Leu Arg Ala Leu Asp Leu Thr Arg Asn Ala Leu Arg
               20                  25                  30
   Ser Leu Pro Pro Gly Leu Phe Ser Thr Ser Ala Asn Leu Ser Thr Leu
           35                  40                  45
   Val Leu Arg Glu Asn Gln Leu Arg Glu Val Ser Ala Gln Trp Leu Gln
       50                  55                  60
   Gly Leu Asp Ala Leu Gly His Leu Asp Leu Ala Glu Asn Gln Leu Ser
   65                  70                  75                  80
   Ser Leu Pro Ser Gly Leu Leu Ala Ser Leu Gly Ala Leu His Thr Leu
                   85                  90                  95
   Asp Leu Gly Tyr Asn Leu Leu Glu Ser Leu Pro Glu Gly Leu Leu Arg
               100                 105                 110
   Gly Pro Arg Arg Leu Gln Arg Leu His Leu Glu Gly Asn Arg Leu Gln
           115                 120                 125
   Arg Leu Glu Asp Ser Leu Leu Ala Pro Gln Pro Phe Leu Arg Val Leu
       130                 135                 140
   Phe Leu Asn Asp Asn Gln Leu Val Gly Val Ala Thr Gly Ser Phe Gln
   145                 150                 155                 160
   Gly Leu Gln His Leu Asp Met Leu Asp Leu Ser Asn Asn Ser Leu Ser
                   165                 170                 175
   Ser Thr Pro Pro Gly Leu Trp Ala Phe Leu Gly Arg Pro Thr Arg Asp
               180                 185                 190
   Met Gln Asp Gly Phe Asp Ile Ser His Asn Pro Trp Ile Cys Asp Lys
           195                 200                 205
   Asn Leu Ala Asp Leu Cys Arg Trp Leu Val Ala Asn Arg Asn Lys Met
       210                 215                 220
   Phe Ser Gln Asn Asp Thr Arg Cys Ala Gly Pro Glu Ala Met Lys Gly
   225                 230                 235                 240
   Gln Arg Leu Leu Asp Val Ala Glu
                   245
<210> 8
   <211> 252
   <212> PRT
   <213> Homo sapiens
<400> 8
   Leu Gln Glu Leu His Leu Ser Ser Asn Gly Leu Glu Ser Leu Ser Pro
   1               5                   10                  15
   Glu Phe Leu Arg Pro Val Pro Gln Leu Arg Val Leu Asp Leu Thr Arg
               20                  25                  30
   Asn Ala Leu Thr Gly Leu Pro Pro Gly Leu Phe Gln Ala Ser Ala Thr
           35                  40                  45
   Leu Asp Thr Leu Val Leu Lys Glu Asn Gln Leu Glu Val Leu Glu Val
       50                  55                  60
   Ser Trp Leu His Gly Leu Lys Ala Leu Gly His Leu Asp Leu Ser Gly
   65                  70                  75                  80
   Asn Arg Leu Arg Lys Leu Pro Pro Gly Leu Leu Ala Asn Phe Thr Leu
                   85                  90                  95
   Leu Arg Thr Leu Asp Leu Gly Glu Asn Gln Leu Glu Thr Leu Pro Pro
               100                 105                 110
   Asp Leu Leu Arg Gly Pro Leu Gln Leu Glu Arg Leu His Leu Glu Gly
           115                 120                 125
   Asn Lys Leu Gln Val Leu Gly Lys Asp Leu Leu Leu Pro Gln Pro Asp
       130                 135                 140
   Leu Arg Tyr Leu Phe Leu Asn Gly Asn Lys Leu Ala Arg Val Ala Ala
   145                 150                 155                 160
   Gly Ala Phe Gln Gly Leu Arg Gln Leu Asp Met Leu Asp Leu Ser Asn
                   165                 170                 175
   Asn Ser Leu Ala Ser Val Pro Glu Gly Leu Trp Ala Ser Leu Gly Gln
               180                 185                 190
   Pro Asn Trp Asp Met Arg Asp Gly Phe Asp Ile Ser Gly Asn Pro Trp
           195                 200                 205
   Ile Cys Asp Gln Asn Leu Ser Asp Leu Tyr Arg Trp Leu Gln Ala Gln
       210                 215                 220
   Lys Asp Lys Met Phe Ser Gln Asn Asp Thr Arg Cys Ala Gly Pro Glu
   225                 230                 235                 240
   Ala Val Lys Gly Gln Thr Leu Leu Ala Val Ala Lys
                   245                 250

## Description

### FIELD OF THE INVENTION

The invention is in the field of molecular physiology and relates to the use of antagonists of Leucine-rich alpha-2-glycoprotein 1 (Lrg1) for use in the treatment or prevention of cancer.

### BACKGROUND OF THE INVENTION

The term cancer relates to a broad range of malignant neoplastic growths, which may arise from the transformation of many normal cell types. Cancer may be associated with many different changes in cell phenotypes. However, all cancers involve unregulated cell growth. There are many different types of anti-cancer drugs. However, many of these are associated with undesirable side effects, often as a result of the non-specific targeting of non-cancerous cells. Also, conventional drugs which are more cancer specific tend to be so for a limited number of particular cancers.

Conventional chemotherapy drugs such as cisplatin act by inhibiting mitosis. These agents are not, however, specific to cancer cells, but instead will affect all rapidly dividing cells. Therefore, non-cancerous but fast-dividing cells, such as cells which replace the intestinal epithelium can often be unintentionally affected.

Similarly, the effect of agents which block growth factor activity, such as tyrosine kinase inhibitors such as imatinib or antibodies to growth factors or their receptors, will not be restricted to neoplastic cells. Agents such as interferon γ (IFNγ) and interleukin 2 (IL2), which can be used to treat kidney cancers, can also affect non-cancerous cells.

Hormone therapy is another common cancer treatment. This type of therapy is only suitable for certain cancers, namely those that are hormone sensitive or hormone dependent. For example, tamoxifen blocks the oestrogen receptor and may be used in the treatment of breast cancer. However, non-cancerous cells which are sensitive to or dependent on the hormone being targeted may also be affected by hormone therapy, leading to undesirable side effects.

Other anti-cancer agents, such as monoclonal antibodies are more specific, but act on only a particular cancer type, for example breast cancer, or even particular subgroups of a cancer. For example, trastuzumab (Herceptin) can only be used to treat HER2 positive cancer, typically HER2 positive breast cancers or stomach adenocarcinomas. An alternative therapeutic approach has been to target the localized immune suppressive environment within the tumour through approaches including gene therapy.

Additionally, for certain cancers, particularly solid tumours, once the cancer grows beyond a certain size, diffusion is no longer sufficient to supply oxygen and nutrients to sustain growth. These tumours must then develop blood vessels, typically via angiogenesis, in order to meet their metabolic needs. Therefore, other conventional anti-cancer drugs include anti-angiogenic agents which impact the development of tumour vasculature, and so limit tumour growth. For example, the anti-VEGF monoclonal antibody bevacizumab (Avastin) is a known anti-angiogenic cancer therapy.

However, not all cancers require the development of tumour vasculature for continued growth. For example myelomas and leukaemias, although even here there are indications that tumour cell proliferation in the bone marrow may be responsive to anti-angiogenics. In addition, even for cancers that do require blood vessel growth, some tumours may not be responsive to anti-angiogenic therapy. Alternatively, it may be desirable to target multiple processes such as tumour angiogenesis, neoplasia and the immune system simultaneously in order to elicit a greater anti-cancer effect.

There is therefore a need to identify alternative therapeutic targets and novel drugs which, in isolation or in combination with existing therapies, may be more effective, suitable for treating a wide range of cancers and possess fewer off-target effects for the treatment of cancer.

### SUMMARY OF THE INVENTION

Leucine-rich alpha-2-glycoprotein 1 (Lrg1 gene identifiers: HGNC: 29480; Entrez Gene: 116844; Ensembl: ENSG00000171236; UniProtKB: P02750) was identified in 1977 (Haupt & Baudner, 1977) and its primary structure determined in 1985 (Takahashi *et al,* 1985). Lrg1 is highly evolutionarily conserved between mice and humans, polyclonal antibodies to human Lrg1 are commercially available and there are reports of concomitant increases in the level of transforming growth factor beta 1 (TGFβ1), TGFβ receptor II (TGFβRII) and Lrg1 in certain diseases (Sun *et al,* 1995; Li *et al,* 1997). Other groups have identified Lrg1 as a biomarker of certain diseases (US 2005/0064516; WO 2008/092214) and as a ligand for cytochrome c (US 2007/0184503). Lynch *et al.* (2012) demonstrate that microRNA-335 (miR-335) targets Lrg1 leading to decreased migration and invasion of neuroblastoma cells by reducing the phosphorylation status of myosin light chain (MLC).

The present inventors have previously shown that Leucine-rich alpha-2-glycoprotein 1 is a drugable target for the modulation of pathogenic vascular remodelling. Therefore, the inventors predicted that antagonising Lrg1 may be useful in the treatment of conditions in which pathogenic vascular remodelling or pathogenic angiogenesis occurs, particularly in the eye in conditions such as neovascular AMD, diabetic retinopathy and retinopathy of prematurity (WO 2011/027129). However, the inventors have now found that Lrg1 has a direct effect on neoplastic cells as well as immune cell function, and so may be used as a target in the treatment and/or prevention of cancer by directly affecting these cells.

The present inventors have now identified Lrg1 as a drugable target for the treatment and/or prevention of cancer. In particular, the inventors have demonstrated that targeting Lrg1 has a direct effect on neoplastic cells, and so targeting Lrg1 can also be used to treat and/or prevent cancer by this direct effect on cancer cells, specifically by down-regulating the proliferation of neoplastic cells, rather than by an effect on tumour vascularisation. They have also found that Lrg1 modifies immune cell properties that contribute to the pro-oncogenic environment.

The inventors have investigated previously the connection between Lrg1 and the TGFβ signalling pathway.

In endothelial cells TGFβ signaling can occur through TGFβ receptor II associating either with the ubiquitous TGFβ type I receptor activin receptor-like kinase 5 (ALK5) or with ALK1, or with ALK5 and ALK1 together, with the cellular response depending on which pathway predominates. In the case of ALK5 there is under certain conditions increased ECM deposition and cell quiescence whilst with ALK1 there is endothelial cell activation manifest as increased migration and proliferation. This differential signalling is partly controlled by the concentration/bioavailability of TGFβ, accessory molecules such as endoglin and betaglycan and by members of a family of downstream effector proteins called Smads, whereby Smad 2 and 3 are activated by ALK5 and Smad 1, 5 and 8 by ALK1. Alternatively, TGFβ receptor activation can activate a non-canonical pathway involving signalling pathways such as Rho GTPase and the MAP kinases.

A further group of proteins known to be important in cancer are the bone morphogenic proteins (BMPs) and their receptors, the bone morphogenic protein receptors (BMPRs). Activin receptor-like kinases (ALKs) can be recruited to BMP/BMPR complexes to mediate signalling within neoplastic cells. The inventors have demonstrated that Lrg1 effects BMP signalling.

Lrg1 has been shown previously by the inventors to form a complex with TGFβRII, ALK5 and ALK1 suggesting that Lrg1 has a role in mediating the formation of this receptor complex and driving signalling down the ALK1/Smad 1, 5 and 8 pathway (WO 2011/027129). One of the mechanisms through which this is achieved is by Lrg1 binding directly to the accessory molecule endoglin which promotes subsequent receptor complex formation. Therefore, the inventors have previously hypothesised that Lrg1 acts as a modulator of TGFβ signalling, causing fine-tuning between the ALK1- and ALK5-activated signalling cascades.

TGFβ is known to play a role in cancer development. During the early stages of tumour progression the TGFß pathway is predominantly suppressive. However, tumour cells are capable of switching their response to TGFß such that it promotes epithelial-mesenchymal transition (EMT), tumour invasion, metastatic dissemination and evasion of the immune system (Meulmeester and ten Dijke., 2011). This is known as the TGFβ switch. Whilst not being bound by this theory, the inventors hypothesise that upregulation of Lrg1 in neoplastic cells causes an alteration in their TGFβ signalling response resulting in a switch from a suppressive to an oncogenic stimulus.

Indeed, in endothelial cells the inventors have found that Lrg1 enhances the mitogenic action of TGFβ. Thus, brain endothelial cells from *Lrg1*^{*-*/*-*} mice proliferated more slowly than those from WT animals. Addition of TGFβ1 significantly enhanced endothelial cell proliferation from WT animals but inhibited the growth of cells from *Lrg1*^{*-*/*-*} mice, presumably through enhanced ALK5-Smad2/3 signalling in the absence of activation of the ALK1-Smad1/5/8 pathway. The addition of Lrg1 on its own had no effect, but when both TGFβ1 and Lrg1 were added cell proliferation increased significantly in both WT and Lrg1 null cells. These observations were further substantiated by studies showing that Lrg1 over-expression in the brain endothelial cell line GPNT led to increased Smad1/5 phosphorylation and enhanced TGFβ1-mediated cell proliferation, whereas Lrg1 knockdown with siRNA resulted in decreased Smad1/5 phosphorylation and reduced cell division. This implies that Lrg1 enhances the mitogenic properties of TGFβ. In support of Lrg1 playing a role in neoplasia, exploration of cancer publications and databases (Table 1) shows that Lrg1 gene and protein expression is frequently increased in tumours such as ovarian, breast, lung and prostate.

In addition to being pro-oncogenic, TGFβ is also known to be immunosuppressive in the tumour environment preventing anti-tumour immunity that supports tumour growth and survival (Flavell et al., 2010). Whilst not being bound by this theory, the inventors also hypothesise that up-regulation of Lrg1 in the tumour environment results in a switch in TGFβ signalling in immune cells causing a shift from anti-tumour immune responses to immune suppression (through mechanisms such as blocking pro-inflammatory T cells, upregulating regulatory immune cells including Tregs and regulatory macrophages, inducing anergy or upregulating tolerogenic mechanisms and promoting activation-induced cell death).

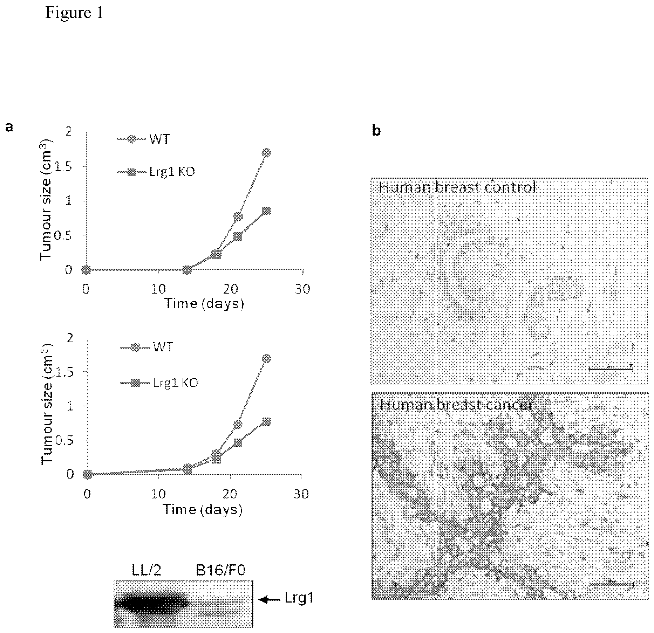

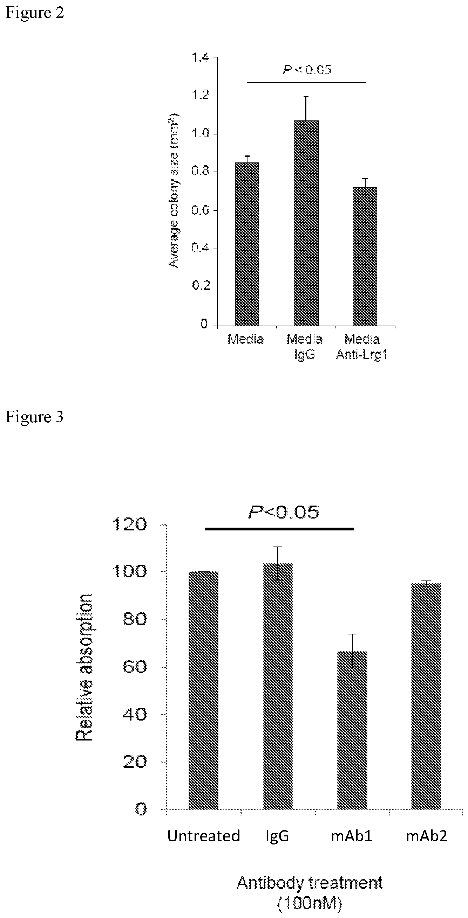

The inventors have shown that in a number of tumours (e.g. breast and glioma) the expression of Lrg1 is greatly up-regulated (Figure 1b) and is expressed in the mouse Lewis lung tumour cell line LL/2 and mouse melanoma cell line B16/F10 (Figure 1a, lower panel). When these cell lines are grafted subcutaneously into wild type C57BL/6 mice and *Lrg1* knockout mice on the same background the growth rate of the tumours is significantly inhibited in the latter (Figure 1a, graphs). These observations suggest that Lrg1 mediates a switch in tumour cell response to TGFß from being suppressive to being pro-oncogenic. In support of this, the inventors have shown that a Lrg1 blocking antibody results in a significant reduction in the size of murine Lewis lung carcinoma (LL/2) cell colonies grown in suspension in an agarose gel (Figure 2)

This idea is further supported by the emerging view that signaling by TGFβ can occur through the TGFβRII/ALK5/ALK2/3-Smad1/Smad5 axis. Thus, recent reports show that in various epithelial cells TGFβ can activate Smad1 and Smad5 through a BMP-independent pathway involving ALK2 and/or ALK3 with ALK5 (Daly et al., 2008).

The inventors have shown that, as well as LL/2 cells, other cell lines secrete Lrg1 including the human mammary cell line MCF10A, the human epithelial lung carcinoma cell line A549 and the human mammary adenocarcinoma cell line MDA MB 468 (Table 2). Moreover, MCF10A and A549 cells also express the accessory receptor endoglin to which Lrg1 has been shown to bind directly.

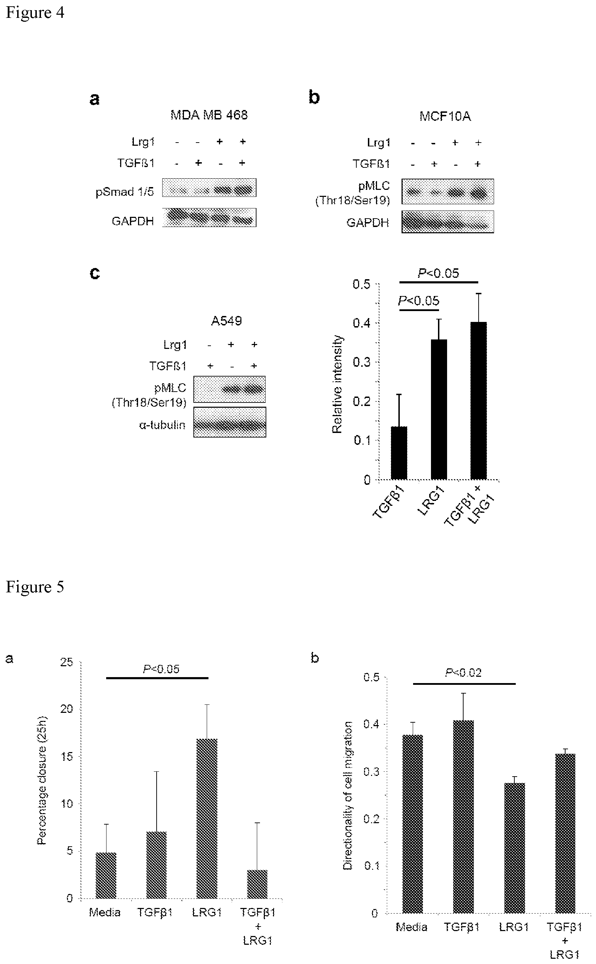

In addition, the inventors have produced evidence to show that Lrg1 can induce both canonical and non-canonical TGFβ signaling in these cells. Thus, they have shown that Lrg1, in the absence of ALK1, induces Smad 1/5 phosphorylation in MDA MB 468 cells (Figure 4a) and myosin light chain phosphorylation (on Thr18/Ser19), indicative of Rho/Rho kinase activation, in MCF10A (Figure 4b) and A549 (Figure 4c) cells. Lrg1 also has an effect on cell migration. Thus, in MCF10A epithelial cells the addition of exogenous Lrg1 causes a significant increase in cell migration as determined by the wound scratch assay (Figure 5a) and a loss of cell migration directionality (Figure 5b) measured over a 5 h time lapse assay.

Having generated mouse monoclonal antibodies against human Lrg1 the inventors show that one such antibody significantly inhibited the rate of proliferation of the human epithelial lung carcinoma cell line A549 (Figure 3). However a second mouse monoclonal anti-Lrg-1 antibody, whilst recognising Lrg1 did not cause any significant reduction in A549 cell proliferation. This establishes the potential of blocking Lrg1 activity in the treatment of cancer.

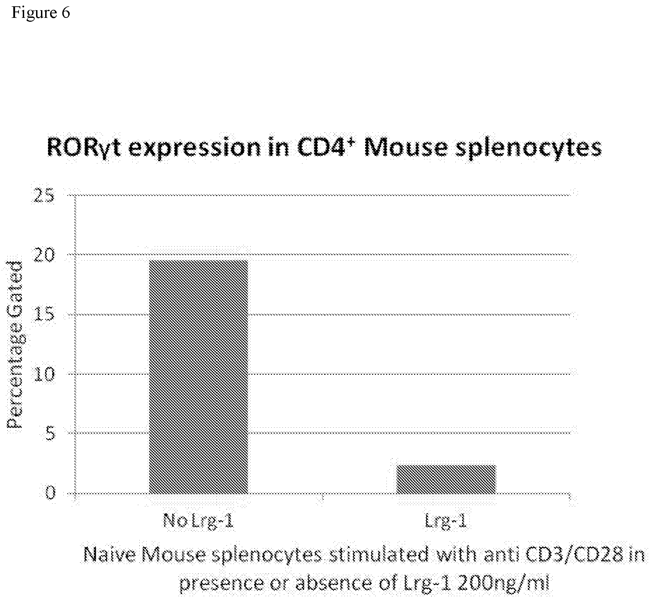

In the immune system the inventors have also shown that expression of the Th17-associated transcription factor ROR_{γt} on CD4 T cells following T cell activation for 5 days with anti-CD3/CD28 stimulation is significantly inhibited in the presence of Lrg1 (Figure 6). This demonstrates that amongst other immune modulating effects Lrg1 has the capacity to induce the suppression of pro-inflammatory T cells. Indeed, it has been proposed that under certain conditions Th17 cells may be anti-tumourigenic and their down-regulation in tumours may support tumour cell survival and expansion (Zou and Restifo., 2010).

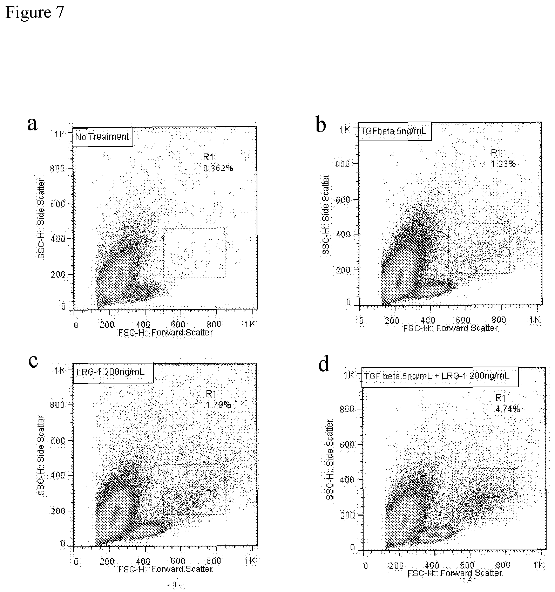

Furthermore, in human peripheral blood mononuclear cells Lrg1 induces a population of CD14/CD11b cells that express the TGFβ accessory receptor endoglin which is a major receptor for Lrg1 (Figure 7) and which alters TGFβ signalling and cell function.

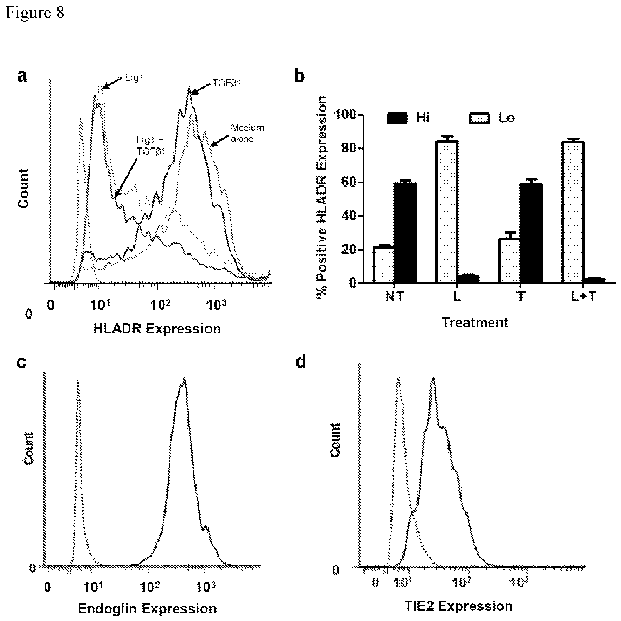

The inventors have also shown that Lrg1-treated monocytes express much less HLA-DR than controls (Figure 8). This is of relevance to cancer because tumour-associated macrophages (TAMs) that express low MHC class II are immunosuppressive and promote tumour angiogenesis. It is also noteworthy that in a gene profiling study investigating differentially expressed genes in TAMs, Lrg1 was found to be up-regulated (Schmieder et al., 2011). A small proportion of TAMs also express Tie2 and low levels of MHC class II, the so-called Tie2 expressing macrophages (TEMs), and deletion of TEMs has been found to greatly improve the efficacy of tumour therapy (Welford *et al,* 2011). The inventors provide data that demonstrate that the ENG^{hi}, HLA-DR^{lo} macrophage phenotype promoted by Lrg1 is also Tie2 positive (Figure 8).

Lrg1 is a potentially superior target to those of conventional anti-cancer agents. Not only is it highly expressed in many cancer cells, increasing its specificity, but also it is expressed by numerous different types of neoplastic cells, suggesting it may be an effective target in treating a wide range of cancers. A further attraction of Lrg1 as a target is that it is extracellular and hence more easily accessed via systemic therapeutic routes.

Accordingly, the invention is defined in the claims and provides:
An antagonist of Leucine-rich alpha-2-glycoprotein 1 (Lrg1) for use in the treatment of a cancer that is not dependent on vasculoproliferation for growth or that is non-responsive to treatment with an anti-angiogenic or anti-vasculoproliferative agent, the antagonist comprising an antibody that binds to Lrg1, a double-stranded RNA that targets Lrg1 RNA, an anti-sense RNA that targets Lrg1 RNA, an aptamer that binds to Lrg1, or a peptide or peptidomimetic that competes with full-length Lrg1 and hence blocks Lrg1 function.

The antagonist preferably acts on non-vascular cells. In a preferred embodiment, the effect of the antagonist of the invention on neoplastic cells is the down-regulation of neoplastic cell proliferation, and may have at least one additional effect on neoplastic cells selected from down-regulation of neoplastic cell migration, down-regulation of cell-cell interactions between neoplastic cells, down-regulation of expression of neoplastic genes by neoplastic cells, and blocking the switch of TGFβ from an anti- to a pro-oncogenic factor for neoplastic cells.

The effect of the Lrg1 antagonist of the invention on neoplastic cells may also be selected from down-regulation of neoplastic cell migration, down-regulation of cell-cell interactions between neoplastic cells, down-regulation of expression of neoplastic genes by neoplastic cells, and blocking the switch of TGFβ from an anti- to a pro-oncogenic factor for neoplastic cells.

The invention further provides an antagonist of Leucine-rich alpha-2-glycoprotein 1 (Lrg1) for use according to the invention, wherein the method of treatment of cancer is by an effect on tumour environment immune cell function. The Lrg1 antagonist may decrease the percentage of CD14 positive CD11b positive cells within a peripheral blood mononuclear cell (PBMC) population compared to a control in which the antagonist is not administered, and/or may increase the percentage of RORγt positive CD4 T cells compared to a control in which the antagonist is not administered.

The Lrg1 antagonist according of the invention may block the interaction between Lrg1 and TGFβ Receptor II (TGFβRII), and/or Lrg1 and TGFβ, and/or Lrg1 and an activin receptor-like kinase (ALK), and/or Lrg1 and endoglin, and/or Lrg1 and betaglycan, and/or Lrg1 and a bone morphogenic protein (BMP), and/or Lrg1 and a bone morphogenic protein receptor (BMPR), and/or an ALK and BMPR in TGFβ or BMP signalling, and/or Lrg1 and activin type II receptor (ACVRII), and/or endoglin and ALK, and/or ALK and BMPR, and/or ALK and TGFβRII. The blocking by an Lrg1 antagonist of the invention may reduce the interaction between endoglin and Lrg1 and thereby modulates the interaction between the ALK and TGFβ Receptor II (TGFβRII), and/or reduce the interaction between betaglycan and Lrg1, and thereby modulates the interaction between the ALK and TGFβRII, and/or disrupt the formation of a BMP, BMPR and ALK complex, or the signalling by said complex, and/or disrupt non-canonical TGFβ signalling, and/or disrupt the formation of a BMP, ACVRII and ALK complex, or the signalling by said complex.

The Lrg1 antagonist is for use in the treatment of a cancer that is not dependent on vasculoproliferation for growth or that is non-responsive to treatment with an anti-angiogenic or anti-vasculoproliferative agent.

The Lrg1 antagonist for use according of the invention may be for use in combination with another anti-cancer therapeutic, which is optionally selected from a cytotoxic agent, a chemotherapeutic agent, a growth inhibitory agent and an anti-cancer monoclonal antibody.

The Lrg1 antagonist for use according of the invention may be for use in combination with an anti-angiogenic compound, which is optionally selected from an antagonist of vascular endothelial growth factor (VEGF), an angiopoietin antagonist, an antagonist of placental growth factor (PLGF), an antagonist of endoglin, a CD160 antagonist or an antagonist of activin receptor-like kinase 1 (ALK1), and preferably said VEGF antagonist is an anti-VEGF antibody.

Disclosed is a method of identifying an antagonist of Lrg1 of the invention comprising:
(a) providing a candidate antagonist, and
(b) determining whether or not said candidate antagonist blocks the direct effect of Lrg1 on neoplastic cells;
wherein said candidate antagonist is identified as an antagonist of Lrg1 if blocking of the effect of Lrg1 on neoplastic cells is observed.

The Lrg1 antagonist may optionally block the interaction between endoglin and Lrg1, and/or Lrg1 and TGFβ Receptor II (TGFβRII), and/or Lrg1 and an activin receptor-like kinase (ALK), and/or Lrg1 and TGFβ, and/or Lrg1 and betaglycan, and/or Lrg1 and a bone morphogenic protein (BMP), and/or Lrg1 and a bone morphogenic protein receptor (BMPR), and/or ALK and BMPR, and/or Lrg1 and activin type II receptor (ACVRII), and/or endoglin and ALK, and/or ALK and BMPR, and/or ALK and TGFβRII.

Disclosed is the use of an antagonist of Lrg1 in the manufacture of a medicament for the treatment or prevention of cancer by an effect on neoplastic cells.

Disclosed is a method of treatment of cancer by an effect on neoplastic cells comprising administering to a patient in need thereof an effective amount of an antagonist of Lrg1.

Disclosed is the use of an antagonist of Lrg1 in the manufacture of a medicament for the treatment or prevention of cancer by an effect on tumour environment immune cell function.

Disclosed is a method of treatment of cancer by an effect on tumour environment immune cell function comprising administering to a patient in need thereof an effective amount of an antagonist of Lrg1.

### BRIEF DESCRIPTION OF THE DRAWINGS

**Figure 1****.** Growth of LL/2 (upper graph) and B16/F10 (lower graph) mouse tumours grafted subcutaneously in wild type and Lrg1-/- mice. Lower panel: Lrg1 western blot of conditioned medium from LL/2 and B16/F10 cell lines. b) Histological sections of normal (top) and cancerous (bottom) human breast tissue stained for Lrg1.
**Figure 2****.** Blocking Lrg1 with a polyclonal anti-Lrg1 antibody reduced the size of LL/2 colonies in an agarose gel. LL/2 cells were suspended at 6.7x10⁴ cells/ml in 0.5% agarose made up in DMEM supplemented with 10% FCS in the presence of 500 nM IgG, 500 nM anti-Lrg1 or neither. The suspension was seeded onto wells that were coated with 1% agarose made up in DMEM. Media and corresponding treatment was added on top of the semisolid suspension and changed weekly. The size of the colonies was analysed after 20 days using ImageJ software.
**Figure 3****.** Addition of one monoclonal anti-Lrg1 antibody, but not a second monoclonal antibody, resulted in a significant reduction in the proliferation of the human lung epithelial carcinoma cell line A549 as assessed by the MTT assay (n≥3). Cells were cultured in DMEM containing 10% FCS over 5 days, with one media change on day 3.
**Figure 4****.** Lrg1 induces canonical and non-canonical TGFβ signalling pathways in normal and tumour-derived epithelial cell lines. Cells were serum starved overnight and then treated with 5 ng/ml TGFβ or 200 ng/ml Lrg1, both in combination or neither for a, b) 60 minutes or c) 10 minutes the following day, before lysing cells for western blot analysis. a) Lrg1 induces Smad 1/5 phosphorylation in the human mammary adenocarcinoma cell line MDA MB 468. b) Lrg1 induces Thr18/Ser19 phosphorylation of myosin light chain (MLC) in the human mammary epithelial cell line MCF10A. c) Lrg1 induces Thr18/Ser19 phosphorylation of myosin light chain (MLC) in the human lung epithelial carcinoma cell line A549. Histogram shows semi-quantification of western blot (n = 3) using ImageJ software.
**Figure 5****.** Invasion and directionality of MCF 10A human mammary epithelial cells in the presence of 5 ng/ml TGFβ or 200 ng/ml Lrg1, both in combination or neither. a) Confluent MCF10A cells were left overnight in appropriate treatment media. "Scratch" wounds were generated using a sterile pipette tip to scrape the cellular monolayer. Fresh media with corresponding treatment conditions was added on top. Analysis of normalised size of scratch was measured over 25 h using ImageJ. The addition of Lrg1 induces a significant increase in the rate of closure of a scratch wound across a monolayer of MCF 10A cells. Concomitant addition of TGFβ reverses the effect (n=3). b) MCF10A cells were seeded at 1x10⁴ cells/ml. The following day they were treated with the appropriate factors. Cell tracks were imaged by time-lapse every 15 min and analysed using ImageJ software, with directionality being a measure of *distance from origin*/*accumulated distance.* Lrg1 causes a significant reduction in the directionality of migration of MCF10A cells (n=3).
**Figure 6****.** Expression of the Th17-associated transcription factor ROR_{γt} on CD4 T cell population following T cell activation for 5 days with anti-CD3/CD28 stimulation in the absence and presence of 200ng/ml Lrg1.
**Figure 7****.** Lrg1/TGFβ induction of an endoglin-positive, CD14/CD11b positive monocytic cell population (boxed region) in human peripheral mononuclear cells. a) untreated cells. b) Following 4 day treatment with 5ng/ml TGFβ. c) Following 4 day treatment with 200ng/ml Lrg1 and d) following 4 day treatment with 5ng/ml TGFβ + 200ng/ml Lrg1.
**Figure 8****.** a) Expression of HLA-DR in human monocytes treated for 48h with medium alone, TGFβ1, Lrg1 or TGFβ1 + Lrg1. b) Comparison of HLADR^{hi} and HLADR^{lo} populations on CD14⁺ macrophages after 48h. NT: No Treatment; L; Lrg-1; T: TGFβ1; L+T: Lrg-1+ TGFβ1. c) HLADR^{lo} CD14⁺ population in presence of Lrg-1 at 48h is endoglin (CD105) positive (solid line). Isotype control (dotted line). d) HLADR¹⁰ CD14⁺ population in presence of Lrg-1 at 48h is TIE2 positive (solid line). Isotype control (dotted line).

### DETAILED DESCRIPTION OF THE INVENTION

### Blocking Lrg1

Antagonists of the invention block the function of Lrg1. Blocking of Lrg1 encompasses any reduction in its activity or function that results in an effect on neoplastic cells or in an effect on tumour environment immune cell function. Effects on neoplastic cells include down-regulating neoplastic cell proliferation, down-regulating neoplastic cell migration and/or migration directionality, modulation of cell-cell interactions between neoplastic cells, down-regulation of expression of neoplastic genes and blocking the switch of TGFβ from an anti- to a pro-oncogenic factor for neoplastic cells. Blocking Lrg-1 may inhibit Lrg-1-induced canonical and/or non-canonical TGFβ signalling. For example, antagonists of the invention may block Lrg-1-mediated ALK1-independent Smad 1/5 phosphorylation, Lrg-1-mediated myosin light chain phosphorylation and/or Lrg-1-mediated Rho/Rho kinase activation. In a preferred embodiment, the effect on neoplastic cells is the down-regulation of neoplastic cell proliferation. Effects on tumour environment immune cell function include decreasing the percentage of CD14 positive CD11b positive cells within a peripheral blood mononuclear cell (PBMC) population and increasing the percentage of ROR_{γt} positive CD4 T cells. Antagonists of the invention may also block a Lrg-1-mediated reduction in MCH class II expression on monocytes and macrophages, particularly tumour-associated macrophages (TAMs). In particular, antagonists of the invention may block a Lrg-1-mediated reduction in HLA-DR expression. Antagonists of the invention may also block a Lrg-1-mediated increase in Tie2 expression on TAMs, particularly Tie2-expressing macrophages (TEMs).

For example, blocking of Lrg1 may be via blocking its interaction with endoglin, betaglycan, an activin receptor-like kinase (ALK), activin type II receptor (ACVRII), TGFβRII and/or TGFβ. Blocking of Lrg1 may also result in reduced bioavailability of TGFβ. Blocking of Lrg1 may involve blocking the interaction between ALK-BMP, ALK-BMPR, endoglin-ALK and/or ALK-TGFβRII. The BMPR is preferably BMPRII.

Blocking encompasses both total and partial reduction of Lrg1 activity or function, for example total or partial prevention of the endoglin-Lrg1, betaglycan-Lrg1, ALK-Lrg1, TGFβRII-Lrg1, ACVRII-Lrg1 and/or TGFβ-Lrg1 interactions. Blocking encompasses both total and partial reduction of Lrg1 activity or function, for example total or partial prevention of the interaction between ALK-BMP, ALK-BMPR, endoglin-ALK and/or ALK-TGFβRII. For example, a blocking antagonist of the invention may reduce the activity of Lrg1 by from 10 to 50%, at least 50% or at least 70%, 80%, 90%, 95% or 99%.

Blocking of Lrg1 activity or function can be measured by any suitable means. For example, blocking of the endoglin-Lrg1, betaglycan-Lrg1, ALK-Lrg1, ACVRII-Lrg1, TGFβRII-Lrg1, TGFβ-Lrg1, ALK-BMP, ALK-BMPR, endoglin-ALK and/or ALK-TGFβRII interaction can be determined by measuring the inhibition of phosphorylation of downstream signalling intermediates. These downstream signalling intermediates may be selected from canonical signalling molecules such as the Smad transcription factors or non-canonical signalling molecules such as MAP kinases, PI3K, Rho GTPase and PKC. The BMPR is preferably BMPRII.

Blocking of Lrg1 can also be measured via assays that measure one of the effects of Lrg1 blockade. For example, proliferation and/or migration studies may be used. Anchorage-independent growth may be used as a measure of tumorigenicity. Lrg1 blockade may also be assessed using in vivo assays such as those that measure rate of tumour growth in animal models in which tumour induction may be achieved by administration of tumour-promoting agents such as phorbol ester, by grafting of tumour cell lines, or in animal models of tumourigenesis such as the RIP-Tag mouse.

Blocking may take place via any suitable mechanism, depending for example on the nature (see below) of the antagonist used, e.g. steric interference in any direct or indirect endoglin-Lrg1, betaglycan-Lrg1, ALK-Lrg1, ACVRII-Lrg1, TGFβRII-Lrg1 TGFβ-Lrg1, ALK-BMP, ALK-BMPR, endoglin-ALK and/or ALK-TGFβRII interaction or knockdown of Lrg1 expression.

### Antagonists of Lrg1

Any suitable antagonist may be used according to the invention, for example peptides and peptidomimetics, antibodies, small molecule inhibitors, double-stranded and antisense RNA, aptamers and ribozymes. Preferred antagonists include peptide fragments of Lrg1, double-stranded RNA, aptamers and antibodies.

### Peptides

Peptide antagonists will typically be fragments of Lrg1 that compete with full-length Lrg1 for binding to TGFβRII, TGFβ, endoglin, betaglycan, BMP, BMPR and/or an activin receptor-like kinase (ALK) and hence antagonise Lrg1. Such peptides may be linear or cyclic. Peptide antagonists will typically be from 5 to 50, preferably 10-40, 10-30 or 15-25 amino acids in length and will generally be identical to contiguous sequences from within Lrg1 but may have less than 100% identity, for example 95% or more, 90% or more or 80% or more, as long as they retain Lrg1-blocking properties. Blocking peptides can be identified in any suitable manner, for example, by systematic screening of contiguous or overlapping peptides spanning part or all of the Lrg1 sequence. Peptidomimetics may also be designed to mimic such blocking peptides.

An peptide antagonist according to the invention may a fragment of Lrg1 having the sequence of L1-24 of Appendix 1 or L94-117 of Appendix 2 (SEQ ID NO: 3), L169-192 of Appendix 1 or L262-285 of Appendix 2 (SEQ ID NO: 4) or L227-252 of Appendix 1 or L320-345 of Appendix 2 (SEQ ID NO: 5) of Lrg1 or a part of any one of these sequences. Alternatively, the peptide antibody of the invention may comprise an amino acid sequence from another region of Lrg1, or a part thereof.

### Double-stranded RNA

Using known techniques and based on a knowledge of the sequence of Lrg1, double-stranded RNA (dsRNA) molecules can be designed to antagonise Lrg1 by sequence homology-based targeting of Lrg1 RNA. Such dsRNAs will typically be small interfering RNAs (siRNAs), usually in a stem-loop ("hairpin") configuration, or micro-RNAs (miRNAs). The sequence of such dsRNAs will comprise a portion that corresponds with that of a portion of the mRNA encoding Lrg1. This portion will usually be 100% complementary to the target portion within the Lrg1 mRNA but lower levels of complementarity (e.g. 90% or more or 95% or more) may also be used.

### Antisense RNA

Using known techniques and based on a knowledge of the sequence of Lrg1, single-stranded antisense RNA molecules can be designed to antagonise Lrg1 by sequence homology-based targeting of Lrg1 RNA. The sequence of such antisense will comprise a portion that corresponds with that of a portion of the mRNA encoding Lrg1. This portion will usually be 100% complementary to the target portion within the Lrg1 mRNA but lower levels of complementarity (e.g. 90% or more or 95% or more) may also be used.

### Aptamers

Aptamers are generally nucleic acid molecules that bind a specific target molecule. Aptamers can be engineered completely *in vitro,* are readily produced by chemical synthesis, possess desirable storage properties, and elicit little or no immunogenicity in therapeutic applications. These characteristics make them particularly useful in pharmaceutical and therapeutic utilities.

As used herein, "aptamer" refers in general to a single or double stranded oligonucleotide or a mixture of such oligonucleotides, wherein the oligonucleotide or mixture is capable of binding specifically to a target. Oligonucleotide aptamers will be discussed here, but the skilled reader will appreciate that other aptamers having equivalent binding characteristics can also be used, such as peptide aptamers.

In general, aptamers may comprise oligonucleotides that are at least 5, at least 10 or at least 15 nucleotides in length. Aptamers may comprise sequences that are up to 40, up to 60 or up to 100 or more nucleotides in length. For example, aptamers may be from 5 to 100 nucleotides, from 10 to 40 nucleotides, or from 15 to 40 nucleotides in length. Where possible, aptamers of shorter length are preferred as these will often lead to less interference by other molecules or materials.

Non-modified aptamers are cleared rapidly from the bloodstream, with a half-life of minutes to hours, mainly due to nuclease degradation and clearance from the body by the kidneys. Such non-modified aptamers have utility in, for example, the treatment of transient conditions such as in stimulating blood clotting. Alternatively, aptamers may be modified to improve their half life. Several such modifications are available, such as the addition of 2'-fluorine-substituted pyrimidines or polyethylene glycol (PEG) linkages.

Aptamers may be generated using routine methods such as the Systematic Evolution of Ligands by Exponential enrichment (SELEX) procedure. SELEX is a method for the *in vitro* evolution of nucleic acid molecules with highly specific binding to target molecules. It is described in, for example, US 5,654,151, US 5,503,978, US 5,567,588 and WO 96/38579.

The SELEX method involves the selection of nucleic acid aptamers and in particular single stranded nucleic acids capable of binding to a desired target, from a collection of oligonucleotides. A collection of single-stranded nucleic acids (e.g., DNA, RNA, or variants thereof) is contacted with a target, under conditions favourable for binding, those nucleic acids which are bound to targets in the mixture are separated from those which do not bind, the nucleic acid-target complexes are dissociated, those nucleic acids which had bound to the target are amplified to yield a collection or library which is enriched in nucleic acids having the desired binding activity, and then this series of steps is repeated as necessary to produce a library of nucleic acids (aptamers) having specific binding affinity for the relevant target.

### Antibodies

The term "antibody" as referred to herein includes whole antibodies and any antigen binding fragment (*i.e.,* "antigen-binding portion") or single chains thereof. An antibody refers to a glycoprotein comprising at least two heavy (H) chains and two light (L) chains inter-connected by disulfide bonds, or an antigen binding portion thereof. Each heavy chain is comprised of a heavy chain variable region (abbreviated herein as V_{H}) and a heavy chain constant region. Each light chain is comprised of a light chain variable region (abbreviated herein as V_{L}) and a light chain constant region. The variable regions of the heavy and light chains contain a binding domain that interacts with an antigen. The V_{H} and V_{L} regions can be further subdivided into regions of hypervariability, termed complementarity determining regions (CDR), interspersed with regions that are more conserved, termed framework regions (FR).

The constant regions of the antibodies may mediate the binding of the immunoglobulin to host tissues or factors, including various cells of the immune system (*e.g.,* effector cells) and the first component (Clq) of the classical complement system.

An antibody of the invention may be a monoclonal antibody or a polyclonal antibody, and will preferably be a monoclonal antibody. An antibody of the invention may be a chimeric antibody, a CDR-grafted antibody, a nanobody, a human or humanised antibody or an antigen binding portion of any thereof. For the production of both monoclonal and polyclonal antibodies, the experimental animal is typically a non-human mammal such as a goat, rabbit, rat or mouse but may also be raised in other species such as camelids.

Polyclonal antibodies may be produced by routine methods such as immunisation of a suitable animal, with the antigen of interest. Blood may be subsequently removed from the animal and the IgG fraction purified.

Monoclonal antibodies (mAbs) of the invention can be produced by a variety of techniques, including conventional monoclonal antibody methodology e.g., the standard somatic cell hybridization technique of Kohler and Milstein. The preferred animal system for preparing hybridomas is the murine system. Hybridoma production in the mouse is a very well-established procedure and can be achieved using techniques well known in the art.

An antibody according to the invention may be produced by a method comprising: immunising a non-human mammal with an immunogen comprising full-length Lrg1, a peptide fragment of Lrg1, an epitope within the sequence of L1-24 of Appendix 1 or L94-117 of Appendix 2 (SEQ ID NO: 3), L169-192 of Appendix 1 or L262-285 of Appendix 2 (SEQ ID NO: 4) or L227-252 of Appendix 1 or L320-345 of Appendix 2 (SEQ ID NO: 5) of Lrg1 or an epitope within other regions of Lrg1; obtaining an antibody preparation from said mammal; and deriving therefrom monoclonal antibodies that specifically recognise said epitope.

The term "antigen-binding portion" of an antibody refers to one or more fragments of an antibody that retain the ability to specifically bind to an antigen. It has been shown that the antigen-binding function of an antibody can be performed by fragments of a full-length antibody. Examples of binding fragments encompassed within the term "antigen-binding portion" of an antibody include a Fab fragment, a F(ab')₂ fragment, a Fab' fragment, a Fd fragment, a Fv fragment, a dAb fragment and an isolated complementarity determining region (CDR). Single chain antibodies such as scFv antibodies are also intended to be encompassed within the term "antigen-binding portion" of an antibody. These antibody fragments may be obtained using conventional techniques known to those of skill in the art, and the fragments may be screened for utility in the same manner as intact antibodies.

An antibody of the invention may be prepared, expressed, created or isolated by recombinant means, such as (a) antibodies isolated from an animal (*e.g.,* a mouse) that is transgenic or transchromosomal for the immunoglobulin genes of interest or a hybridoma prepared therefrom, (b) antibodies isolated from a host cell transformed to express the antibody of interest, *e.g.,* from a transfectoma, (c) antibodies isolated from a recombinant, combinatorial antibody library, and (d) antibodies prepared, expressed, created or isolated by any other means that involve splicing of immunoglobulin gene sequences to other DNA sequences.

An antibody of the invention may be a human antibody or a humanised antibody. The term "human antibody", as used herein, is intended to include antibodies having variable regions in which both the framework and CDR regions are derived from human germline immunoglobulin sequences. Furthermore, if the antibody contains a constant region, the constant region also is derived from human germline immunoglobulin sequences. The human antibodies of the invention may include amino acid residues not encoded by human germline immunoglobulin sequences (*e.g.,* mutations introduced by random or site-specific mutagenesis *in vitro* or by somatic mutation *in vivo).* However, the term "human antibody", as used herein, is not intended to include antibodies in which CDR sequences derived from the germline of another mammalian species, such as a mouse, have been grafted onto human framework sequences.

Such a human antibody may be a human monoclonal antibody. Such a human monoclonal antibody may be produced by a hybridoma which includes a B cell obtained from a transgenic nonhuman animal, *e.g.,* a transgenic mouse, having a genome comprising a human heavy chain transgene and a light chain transgene fused to an immortalized cell.

Human antibodies may be prepared by *in vitro* immunisation of human lymphocytes followed by transformation of the lymphocytes with Epstein-Barr virus.

The term "human antibody derivatives" refers to any modified form of the human antibody, e.g., a conjugate of the antibody and another agent or antibody.

The term "humanized antibody" is intended to refer to antibodies in which CDR sequences derived from the germline of another mammalian species, such as a mouse, have been grafted onto human framework sequences. Additional framework region modifications may be made within the human framework sequences.

Screening methods as described herein may be used to identify suitable antibodies that are capable of binding to Lrg1. Thus, the screening methods described herein may be carried out using an antibody of interest as the test compound.

Antibodies of the invention can be tested for binding to Lrg1 by, for example, standard ELISA or Western blotting. An ELISA assay can also be used to screen for hybridomas that show positive reactivity with the target protein. The binding specificity of an antibody may also be determined by monitoring binding of the antibody to cells expressing the target protein, for example by flow cytometry. Thus, a screening method of the invention may comprise the step of identifying an antibody that is capable of binding Lrg1 by carrying out an ELISA or Western blot or by flow cytometry. Antibodies having the required binding properties may then be further tested to determine their effects on the activity of Lrg1 as described further above.

Antibodies of the invention will have Lrg1 antagonist (blocking) properties as discussed above. In one embodiment, a monoclonal antibody specifically recognises an epitope within Lrg1 and blocks the activity of Lrg1. In one embodiment, the monoclonal antibody specifically recognises an epitope within Lrg1 and blocks the interaction between TGFβRII, TGFβ, an ALK, endoglin, betaglycan, a BMP or a BMPR and Lrg1. In one embodiment, a monoclonal antibody specifically recognises an epitope within amino acids L1-24 of Appendix 1 or L94-117 of Appendix 2 (SEQ ID NO: 3), L169-192 of Appendix 1 or L262-285 of Appendix 2 (SEQ ID NO: 4) or L227-252 of Appendix 1 or L320-345 of Appendix 2 (SEQ ID NO: 5) and blocks the activity of Lrg1. In one embodiment, a monoclonal antibody specifically recognises an epitope within amino acids L1-24 of Appendix 1 or L94-117 of Appendix 2 (SEQ ID NO: 3), L169-192 of Appendix 1 or L262-285 of Appendix 2 (SEQ ID NO: 4) or L227-252 of Appendix 1 or L320-345 of Appendix 2 (SEQ ID NO: 5) and blocks the interaction between TGFβRII, TGFβ, an ALK, endoglin, betaglycan, a BMP, BMPRII or activin type II receptor (ACVRII) and Lrg1.

Antibodies of the invention specifically recognise Lrg1, i.e. epitopes within Lrg1. An antibody, or other compound, "specifically binds" or "specifically recognises" a protein when it binds with preferential or high affinity to the protein for which it is specific but does not substantially bind, or binds with low affinity, to other proteins. The specificity of an antibody of the invention for target protein may be further studied by determining whether or not the antibody binds to other related proteins as discussed above or whether it discriminates between them. For example, an antibody of the invention may bind to human Lrg1 but not to mouse or other mammalian Lrg1.

Antibodies of the invention will desirably bind to Lrg1 with high affinity, preferably in the picomolar range, e.g. with an affinity constant (K_{D}) of 10nM or less, 1nM or less, 500pM or less or 100pM or less, measured by surface plasmon resonance or any other suitable technique.

Once a suitable antibody has been identified and selected, the amino acid sequence of the antibody may be identified by methods known in the art. The genes encoding the antibody can be cloned using degenerate primers. The antibody may be recombinantly produced by routine methods.

Epitopes within Lrg1 can be identified by methods known in the art and discussed herein, notably by systematic screening of contiguous or overlapping peptides via a "PEPSCAN" approach or by forming antibodies to peptide fragments (see above) shown to block Lrg1. Examples of such peptides within which epitopes can be identified for antibody production are the L1-24 of Appendix 1 or L94-117 of Appendix 2 (SEQ ID NO: 3), L169-192 of Appendix 1 or L262-285 of Appendix 2 (SEQ ID NO: 4) and L227-252 of Appendix 1 or L320-345 of Appendix 2 (SEQ ID NO: 5) peptides discussed herein. These and other epitope-containing peptides can be used as immunogens for the generation of antibodies.

### Effect on neoplastic cells

As discussed herein, the inventors have previously shown that Lrg1 antagonists can be used to inhibit angiogenesis. Anti-angiogenic agents are a known class of anti-cancer therapeutics. However, the present inventors have now shown that Lrg1 antagonists can exert an effect on neoplastic cells, i.e. that Lrg1 antagonists are capable of having an anti-cancer therapeutic effect that is independent of the ability of these antagonists to inhibit angiogenesis.

The effect of the Lrg1 antagonists of the invention on neoplastic cells may be direct, i.e. the antagonist interacts directly with one or more neoplastic cells, or indirect, i.e. the antagonist interacts with another cell type or compound and exerts its effect on neoplastic cells via an intermediary. Preferably the effect of the Lrg1 antagonist on neoplastic cells is direct.

Effects of Lrg1 antagonists on neoplastic cells include the down-regulation of neoplastic cell proliferation, down-regulation of epithelial-mesenchymal transition (EMT), tumour invasion, metastatic dissemination, evasion of the immune system, down-regulation of neoplastic cell migration, modulation, including down-regulation, of cell-cell interactions between neoplastic cells, down-regulation of neoplastic gene (such as onocgenes) expression or up-regulation of anti-neoplastic gene expression (such as tumour suppressor genes) by neoplastic cells or blocking the switch of TGFβ from an anti- to a pro-oncogenic factor for neoplastic cells.

The Lrg1 antagonist of the invention may reduce the activity of Lrg1 such that, for example the proliferation, migration, cell-cell interaction, neoplastic gene expression or TGFβ switch is reduced by from 10 to 50%, at least 50% or at least 70%, 80%, 90%, 95% or 99%. If the effect of the Lrg1 antagonist is to increase the expression of anti-neoplastic genes by a neoplastic cell, the antagonist may increase this expression by from 10 to 50%, at least 50%, at least 70%, at least 80%, at least 90% or at least double the expression of the anti-neoplastic genes in the absence of the antagonist.

The effect of the Lrg1 antagonist may be mediated by the antagonist blocking the interaction between endoglin-Lrg1, betaglycan-Lrg1, TGFβRII-Lrg1, TGFβ-Lrg1, an activin receptor-like kinase (ALK)-Lrg1, a bone morphogenic protein (BMP)-Lrg1, a bone morphogenic protein receptor (BMPR)-Lrg1, BMP-ALK, BMPR-ALK, activin type II receptor (ACVRII)-Lrg1, endoglin-ALK or ALK- TGFβRII. The bone morphogenic protein receptor is preferably BMPRII.

The effect of the Lg1 antagonist of the invention on neoplastic cells can either be measured directly, for example measuring neoplastic cell proliferation, migration and/or migration directionality in the presence of the antagonist (and optionally comparing this with the level of neoplastic cell proliferation, migration and/or migration directionality in the absence of the antagonist), or by measuring the level of interaction between endoglin-Lrg1, betaglycan-Lrg1, TGFβRII-Lrg1, TGFβ-Lrg1, an activin receptor-like kinase (ALK)-Lrg1, a bone morphogenic protein (BMP)-Lrg1, a bone morphogenic protein receptor (BMPR)-Lrg1, BMP-ALK or BMPR-ALK, activin type II receptor (ACVRII)-BMPR, ACVRII-Lrg1, endoglin-ALK or ALK-TGFβRII or the presence of downstream signalling molecules. Standard techniques are known in the art for measuring such parameters.

The present inventors' work suggests that endoglin may be a Lrg1 receptor also that Lrg1 can be mitogenic in certain cells. Therefore, Lrg1 may exert a proliferative effect via cells expressing endoglin. Further, TGFβ is known to have mitogenic properties and the inventors have shown previously that Lrg1 modulates TGFβ signalling. Therefore, Lrg1 antagonists may be able to exert an anti-cancer effect on neoplastic cells indirectly, by inhibiting the proliferative effect of Lrg1 brought about via endoglin and TGFβ.

### Therapeutic Indications

Any cancer in which Lrg-1 mediates an effect on neoplastic cells or on tumour environment immune cell function may in principle be treated, prevented or ameliorated according to the present disclosure. An "effect on neoplastic cells" or similar terms as used herein encompass any and all direct effects of Lrg1 on neoplastic cells, including effects on neoplastic cell proliferation, neoplastic cell migration, neoplastic cell adhesion to other neoplastic cells, expression of neoplastic genes or genes required for cancer growth and progression, the switch of TGFβ from an anti- to a pro-oncogenic factor. An "effect on tumour environment immune cell function" or similar terms as used herein encompass any and all effects of Lrg1 on any immune cells in proximity with the tumour, i.e. in direct or indirect contact within neoplastic cells or within the boundaries of neoplastic growth, including decreasing the percentage of CD14 positive CD11b positive cells within a peripheral blood mononuclear cell (PBMC) population or increasing the percentage of RORγt positive CD4 T cells and through mechanisms on the immune system such as blocking pro-inflammatory T cells, upregulating regulatory immune cells including Tregs and regulatory macrophages, inducing anergy or upregulating tolerogenic mechanisms and promoting activation-induced cell death. In the context of the present invention, the term "immune cells" or any similar term used herein encompass both polymorphonuclear leukocytes and mononuclear leukocyes. The polymorphonuclear leukocytes may be one or more of neutrophils, eosinophils and basophils. The mononuclear leukocytes may be one or more of B lymphocytes, T lymphocytes, monocytes, macrophages and dendritic cells. In a preferred embodiment the immune cells are mononuclear leukocytes. In a particularly preferred embodiment the mononuclear leukocytes are monocytes or T lymphocytes. In an even more preferred embodiment, the immune cells are CD4 positive (T helper lymphocytes).

All effects of the Lrg1 antagonist of the invention, alone or in combination with another anti-cancer therapeutic, may be measured in comparison with an appropriate control in which the Lrg1 antagonist or, where appropriate, combination of Lrg1 antagonist and another anti-cancer therapeutic, have not been administered.

Tumours in which Lrg1-mediated effects occur but which are not reliant on angiogenesis for tumour growth are conditions which are be treated, prevented or ameliorated according to the present invention. Also, tumours which are non-responsive or resistant to anti-angiogenic therapeutics are treated, prevented or ameliorated according to the present invention. Further, tumours which may benefit from the inhibition of both tumour angiogenesis and the inhibition of the direct effects of Lrg1 on neoplastic cells may be treated, prevented or ameliorated according to the present invention.

Preferably, there is no, or minimal effect on normal cells, even when the normal cells are in close proximity or direct contact with the tumour cells to be treated.

Tumours that may be treated, prevented or ameliorated according to the present invention include myeloma, leukaemia, brain, breast, kidney, colorectal, lung, prostate, head and neck, stomach, pancreatic, skin, cervical, bone, ovarian, testicular and liver tumours.

### Pharmaceutical Compositions, Dosages and Dosage Regimes

Antagonists of the invention will typically be formulated into pharmaceutical compositions, together with a pharmaceutically acceptable carrier.

As used herein, "pharmaceutically acceptable carrier" includes any and all solvents, dispersion media, coatings, antibacterial and antifungal agents, isotonic and absorption delaying agents, and the like that are physiologically compatible. Preferably, the carrier is suitable for parenteral, e.g. intravenous, intramuscular, subcutaneous, intraocular or intravitreal administration (*e.g.,* by injection or infusion). Depending on the route of administration, the modulator may be coated in a material to protect the compound from the action of acids and other natural conditions that may inactivate the compound.

The pharmaceutical compounds of the invention may include one or more pharmaceutically acceptable salts. A "pharmaceutically acceptable salt" refers to a salt that retains the desired biological activity of the parent compound and does not impart any undesired toxicological effects. Examples of such salts include acid addition salts and base addition salts.

Preferred pharmaceutically acceptable carriers comprise aqueous carriers or diluents. Examples of suitable aqueous carriers that may be employed in the pharmaceutical compositions of the invention include water, buffered water and saline. Examples of other carriers include ethanol, polyols (such as glycerol, propylene glycol, polyethylene glycol, and the like), and suitable mixtures thereof, vegetable oils, such as olive oil, and injectable organic esters, such as ethyl oleate. In many cases, it will be preferable to include isotonic agents, for example, sugars, polyalcohols such as mannitol, sorbitol, or sodium chloride in the composition.

Therapeutic compositions typically must be sterile and stable under the conditions of manufacture and storage. The composition can be formulated as a solution, microemulsion, liposome, or other ordered structure suitable to high drug concentration.

Pharmaceutical compositions of the invention may comprise additional active ingredients as discussed herein.

Disclosed are kits comprising antagonists of the invention and instructions for use. The kit may further contain one or more additional reagents, such as an additional therapeutic or prophylactic agent as discussed below.

The antagonists and compositions of the present invention may be administered for prophylactic and/or therapeutic treatments.

In therapeutic applications, modulators or compositions are administered to a subject already suffering from a disorder or condition as described above, in an amount sufficient to cure, alleviate or partially arrest the condition or one or more of its symptoms. Such therapeutic treatment may result in a decrease in severity of disease symptoms, or an increase in frequency or duration of symptom-free periods. An amount adequate to accomplish this is defined as a "therapeutically effective amount".

In prophylactic applications, formulations are administered to a subject at risk of a disorder or condition as described above, in an amount sufficient to prevent or reduce the subsequent effects of the condition or one or more of its symptoms. An amount adequate to accomplish this is defined as a "prophylactically effective amount". Effective amounts for each purpose will depend on the severity of the disease or injury as well as the weight and general state of the subject.

A subject for administration of the antagonists of the invention may be a human or non-human animal. The term "non-human animal" includes all vertebrates, e.g., mammals and non-mammals, such as non-human primates, sheep, dogs, cats, horses, cows, chickens, amphibians, reptiles, etc. Administration to humans is preferred.

An antagonist of the present invention may be administered via one or more routes of administration using one or more of a variety of methods known in the art. As will be appreciated by the skilled artisan, the route and/or mode of administration will vary depending upon the desired results. Preferred routes of administration for modulators of the invention include intravenous, intramuscular, intradermal, intraocular, intraperitoneal, subcutaneous, spinal or other parenteral routes of administration, for example by injection or infusion, intra-cranial ventricular or sub-dural administration. The phrase "parenteral administration" as used herein means modes of administration other than enteral and topical administration, usually by injection. Alternatively, an antibody of the invention can be administered via a non-parenteral route, such as a topical, epidermal or mucosal route of administration.

A suitable dosage of an antagonist of the invention may be determined by a skilled medical practitioner. Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

A suitable dose may be, for example, in the range of from about 0.1 µg/kg to about 100mg/kg body weight of the patient to be treated. For example, a suitable dosage may be from about 1µg/kg to about 10mg/kg body weight per day or from about 10 g/kg to about 5 mg/kg body weight per day.

Dosage regimens may be adjusted to provide the optimum desired response (*e.g.,* a therapeutic response). For example, a single dose may be administered, several divided doses may be administered over time or the dose may be proportionally reduced or increased as indicated by the exigencies of the therapeutic situation. Dosage unit form as used herein refers to physically discrete units suited as unitary dosages for the subjects to be treated; each unit contains a predetermined quantity of active compound calculated to produce the desired therapeutic effect in association with the required pharmaceutical carrier.

Administration may be in single or multiple doses. Multiple doses may be administered via the same or different routes and to the same or different locations. Alternatively, doses can be via a sustained release formulation, in which case less frequent administration is required. Dosage and frequency may vary depending on the half-life of the antagonist in the patient and the duration of treatment desired.

As discussed in detail below, antagonists of the invention may be co-administered with one or other more other therapeutic agents.

### Combination Therapies

As noted above, Lrg1 antagonists of the invention may be administered in combination with any other suitable active compound. In particular, the Lrg1 antagonist of the invention may be administered in combination with one or more additional anti-cancer therapeutics and/or one or more anti-angiogenic agents. Combination therapy includes administration of a single pharmaceutical dosage formulation which contains Lrg1 antagonist of the invention and one or more additional therapeutic agents; as well as administration of a Lrg1 antagonist of the invention and one or more additional therapeutic agent(s) in its own separate pharmaceutical dosage formulation. For example, a Lrg1 antagonist of the invention and a cytotoxic agent, a chemotherapeutic agent, a growth inhibitory agent or an anti-cancer monoclonal antibody can be administered to the patient together in a single dosage composition such as a combined formulation, or each agent can be administered in a separate dosage formulation. Where separate dosage formulations are used, the Lrg1 antagonist of the invention and one or more additional therapeutic agents can be administered concurrently, or at separately staggered times, i.e., sequentially. The anti-cancer effect exerted by the combination of a Lrg1 antagonist and another anti-cancer therapeutic will preferably be greater than the anti-cancer effect of either the Lrg1 antagonist or the other anti-cancer therapeutic administered alone.

The term "cytotoxic agent" as used herein refers to a substance that inhibits or prevents the function of cells and/or causes destruction of cells. The term is intended to include radioactive isotopes (for example I¹³¹, I¹²⁵, Y⁹⁰ and Re¹⁸⁶), chemotherapeutic agents, and toxins such as enzymatically active toxins of bacterial, fungal, plant or animal origin, or fragments thereof.

A "chemotherapeutic agent" is a chemical compound useful in the treatment of cancer. Examples of chemotherapeutic agents include alkylating agents such as thiotepa and cyclosphosphamide (Cytoxan®); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamine; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as aclacinomysins, actinomycin, authramycin, azaserine, bleomycins, cactinomycin, calicheamicin, carabicin, carminomycin, carzinophilin, chromomycins, dactinomycin, daunorubicin, detorubicin, 6-diazo-5-oxo-L-norleucine, doxorubicin, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; antimetabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elfomithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; PSK®; razoxane; sizofiran; spirogermanium; tenuazonic acid; triaziquone; 2, 2',2"-trichlorotriethylamine; urethan; vindesine; dacarbazine; mannomustine; mitobronitol; mitolactol; pipobroman; gacytosine; arabinoside ("Ara-C"); cyclophosphamide; thiotepa; taxanes, e.g. paclitaxel (Taxol®, Bristol-Myers Squibb Oncology, Princeton, N.J.) and docetaxel (Taxotere®; Aventis Antony, France); gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; xeloda; ibandronate; CPT-11 ; topoisomerase inhibitor RFS 2000; difluoromethylornithine (DMFO); retinoic acid; esperamicins; capecitabine; and pharmaceutically acceptable salts, acids or derivatives of any of the above. Also included in this definition are anti-hormonal agents that act to regulate or inhibit hormone action on tumours such as anti-oestrogens including for example tamoxifen, raloxifene, aromatase inhibiting 4(5)-imidazoles, 4-hydroxytamoxifen, trioxifene, keoxifene, LY 117018, onapristone, and toremifene (Fareston); and antiandrogens such as flutamide, nilutamide, bicalutamide, leuprolide, and goserelin; and pharmaceutically acceptable salts, acids or derivatives of any of the above.

A "growth inhibitory agent" when used herein refers to a compound or composition which inhibits growth of a cell, especially a cancer cell either *in vitro* or *in vivo.* Examples of growth inhibitory agents include agents that block cell cycle progression (at a place other than S phase), such as agents that induce G1 arrest and M-phase arrest. Classical M-phase blockers include the vincas (vincristine and vinblastine), Taxol ®, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C.

An "anti-cancer monoclonal antibody" when used herein means any monoclonal antibody that can be used to exert an anti-cancer effect, regardless of the mechanism by which that anti-cancer effect is achieved. Examples of anti-cancer monoclonal antibodies include alemtuzumab (Campath) for the treatment of chronic lymphocytic leukemia, bevacizumab (Avastin) for the treatment of brain cancer, colon cancer, kidney cancer and lung cancer, cetuximab (Erbitux) for the treatment of colon cancer and head and neck cancers, ibritumomab (Zevalin) for the treatment of non-Hodgkin's lymphoma, ofatumumab (Arzerra) for the treatment of chronic lymphocytic leukemia, panitumumab (Vectibix) for the treatment of colon cancer, rituximab (Rituxan) for the treatment of chronic lymphocytic leukaemia and non-Hodgkin's lymphoma, tositumomab (Bexxar) for the treatment of non-Hodgkin's lymphoma, trastuzumab (Herceptin) for the treatment of breast cancer and stomach cancer and edrecolomab (Panorex) for the treatment of colon cancer.

Lrg1 antagonists of the invention may be administered in combination with one or more anti-angiogenic agent or compound. The anti-angiogenic compound may be selected an antagonist of any pro-angiogenic molecule. Antagonists may be selected from suitable peptides and peptidomimetics, antibodies, small molecule inhibitors, double-stranded RNA, aptamers and ribozymes, as discussed above in relation to Lrg1. For example, the anti-angiogenic compound may be selected from an antagonist of vascular endothelial growth factor (VEGF), an angiopoietin antagonist, an antagonist of placental growth factor (PLGF), an antagonist of endoglin, a CD160 antagonist or an antagonist of activin receptor-like kinase 1 (ALK1). Preferably the anti-angiogenic agent is a VEGF or PLGF antagonist. VEGF antagonists may preferably be an anti-VEGF antibody such as Avastin and/or Lucentis and/or a receptor-based VEGF trap such as Aflibercept.

The following Examples illustrate the invention.

### EXAMPLES

### 1. Tumour growth is decreased in Lrg1 knock-out mice

The effect of Lrg1 on tumour growth was investigated. B16-F0 (melanoma) or LL/2 (Lewis lung carcinoma) cells were grown as adhesion cultures in DMEM supplemented with 10% FCS, 4mM L-glutamine and 100U/ml penicillin and 100µg/ml streptomycin. Subconfluent cells were trypsinised and resuspended in serum-free medium. 1x10⁶ tumour cells in 100µl were injected subcutaneously into the dorsal region near the thigh of 2 month old wild-type (WT) or Lrg1 knock-out (KO) mice. Tumours were calipered 3 times a week 7 days after injection. Mice were sacrificed when the tumour had reached a volume exceeding 1.5cm³. At the end of the experiment, tumours were harvested and processed for histological analysis.

For both the B16-F0 and LL/2 tumours, tumour growth was decreased in Lrg1 KO mice compared to in WT mice (Figure 1a). The data presented in Figure 1a actually underestimates the difference in tumour growth between WT and Lrg1 KO mice, because tumours in WT mice often reached the end volume of 1,5cm³, and so had to be sacrificed, before the end point of the experiment. These data show that in mice lacking endogenous Lrg1 expression tumour growth, i.e. the total number of tumour cells, was decreased. This demonstrates that Lrg1 stimulates tumour cell proliferation.

Culture medium from samples of the B16-F0 and LL/2 cell lines were analysed by Western blotting and both cancer cell lines were found to express and secrete Lrg1 protein (Figure 1a, bottom panel). Tissue arrays from a human breast cancer (derived from breast epithelial cells) and a control of non-cancerous human breast tissue were subjected to histological analysis to examine Lrg1 expression (Figure 1b). An antibody specific for Lrg1 was used to stain the arrays, with Lrg1 expression being indicated by brown staining of the sample. The non-cancerous breast tissue expressed little Lrg1, with only minimal staining being observed in epithelial cells (Figure 1b, top panel). However, the human breast cancer tissue array stained strongly for Lrg1 (Figure 1b, bottom panel). The most strongly stained cells are thought to be expressing Lrg1 and secreting it into the extracellular environment of the tumour, hence the background level of Lrg1 staining observed across the breast cancer array (almost no background staining is observed in the control sample). This secreted Lrg1 could then not only act on other tumour cells, but also on other cells in the tumour environment, such as immune cells.

From these Western blot and histological data it was concluded that tumour cells are capable of producing their own supply of Lrg1. As tumour growth has been shown to be reduced in the absence of Lrg1 (Figure 1a), it is predicted that in the absence of this tumour-generated Lrg1, or if the biological activity of the Lrg1 produced by the tumour cells were blocked, the level of tumour cell proliferation and tumour growth would be even further reduced in Lrg1 KO mouse. This could be achieved because there is no Lrg1 produced by the KO mouse and the activity of Lrg1 that is produced by the tumour cells would be inhibited.

### 2. Lrg1 expression is up-regulated in a variety of tumours

Having found that tumour growth is decreased in the absence of Lrg1 expression, the inventors then investigated the expression of Lrg1 in a variety of tumour types. Exploration of cancer publications and databases (Table 1) shows that Lrg1 gene and protein expression is frequently increased in tumours such as ovarian, breast, lung and prostate. Taken together with the results in Example 1, this demonstrates that Lrg1 is a drugable target for the treatment of cancer.

### 3. Anti-Lrg1 antibodies reduce tumour cell growth in vitro

Having established that Lewis Lung carcinoma (LL/2) cells grow more slowly in Lrg1^{-/-} mice compared to WT controls we next determined whether Lrg1 blockade with an anti-Lrg1 polyclonal antibody reduced anchorage independent cell growth using a standard soft agar colony formation assay. LL/2 cells were suspended at 6.7 x 10⁴ cells/ml in 0.5% agarose made up in DMEM supplemented with 10% FCS in the presence of 500 nM IgG, 500 nM anti-Lrg1 polyclonal antibody or with media alone. The suspension was seeded onto wells that were coated with 1% agarose made up in DMEM. Media and corresponding treatment was added on top of the semi-solid suspension and changed weekly. The size of the colonies were analysed after 20 days using ImageJ software. Blockade of Lrg1 resulted in a significant reduction in colony size compared to media alone or irrelevant IgG controls (Figure 2).

Having generated monoclonal antibody (mAb) against human LRG1 we next determined whether any of these antibodies could block the proliferation of the human lung epithelial carcinoma cell line A549. Addition of one monoclonal, but not a second, resulted in a significant reduction in the in vitro proliferation of the A549 cells as assessed by the MTT assay (n = 3) (Figure 3). In this experiment the culture media were supplemented with 100nM mAb and the cells were maintained in culture for 5 days. The result shows that functional blockade of Lrg1 leads to a reduction in cell growth rate, further supporting the idea that targeting Lrg1 in cancer may have therapeutic value.

### 4. Lrg1 induces canonical and non-canonical TGFβ signalling

To determine whether Lrg1 can induce canonical or non-canonical TGFβ signalling pathways in normal and tumour-derived epithelial cell lines cells were treated in vitro with various combinations of Lrg1 and TGFβ1. Cells were serum starved overnight and then treated with 5 ng/ml TGFβ or 200 ng/ml Lrg1 or a combination of both and then after 10-60 min were lysed and analysed by western blot. In the human mammary adenocarcinoma cell line MDA MB 468, Lrg1 was found to induce Smad 1/5 phosphorylation (Figure 4a) indicating that the canonical signalling pathway can be induced. Moreover, in both the human mammary epithelial cell line MCF10A and the human lung epithelial carcinoma cell line A549, Lrg1 was found to induce non-canonical activation of the Rho/ROCK pathway as revealed by myosin light chain (MLC) Thr18/Ser19 phosphorylation (Figure 4b and c) which is consistent with activation of EMT. The histogram shows semi-quantification of western blot for the A549 cells (n = 3) using ImageJ software.

### 5. Lrg1 induces canonical and non-canonical TGFβ signalling

As Lrg1 was shown to induce both canonical and non-canonical signalling in these cells we next determined whether this resulted in a change in cell function. The migratory function of MCF 10A human mammary epithelial cells was evaluated in media alone or in the presence of 5 ng/ml TGFβ, 200 ng/ml Lrg1 or a combination of both factors. To determine migration we employed the widely-used scratch (wound closure) assay where a scratch was made through a confluent monolayer of MCF10A cells with a sterile pipette tip. Fresh media with corresponding treatments was added and the closure of the scratch by migrating cells was recorded over a 25 h period. The scratch size was then measured using ImageJ. Addition of Lrg1 was found to induce a significant increase in the rate of closure compared to untreated cells (Figure 5a). TGFβ alone did not alter the closure rate but in combination with Lrg1 reversed the effect (n=3). The directionality of cell migration was then determined in MCF10A cells. Cells were seeded at low confluency (1x10⁴ cells/ml) and treated as described above. Cell tracks were imaged by time-lapse microscopy every 15 min and analysed using Image J software, with directionality being a measure of distance from origin/accumulated distance. Analysis revealed that Lrg1 causes a significant reduction in the directionality of migration of MCF10A cells (n=3) (Figure 5b). These data indicate that in addition to inducing cell signalling Lrg1 also affects cell behaviour.

### 6. Lrg1 expression modulates immune cell populations

Spleens from C57/BL6 mice were made into single cell suspensions and counted on a hemocytometer. Naive T cells were isolated from 1 x 10⁸ cells using the CD4⁺ CD62L⁺ T cell isolation kit (Miltenyl Biotech). Cells were cultured in 96 well plates at a density of 1 x 10⁶/ml and stimulated with 1µg/ml soluble anti CD3 and 2µg/ml soluble anti CD28 with and without addition of 200ng/ml recombinant human LRG-1 and maintained at 37°C under 5% CO₂. After 5 days cells were surface stained for CD4 (APC) and intracellular stained for nuclear transcription factors ROR_{γt} (PE) and FoxP3 (FITC). Data were acquired on a FACSCalibur and analysed using FlowJo software.

Treatment of the mouse splenocytes with Lrg1 reduced the population of Th17 CD4+ T cells from nearly 20% to less than 5% (Figure 6). These data therefore demonstrate that Lrg1 is able to modulate immune cells. In particular, the inventors have shown that Lrg1 modulates the mouse splenocytes to reduce a population of T cells that could attack cancer cells if those T cells were present.

Human peripheral blood mononuclear cells (PBMCs) were isolated from the whole blood of healthy donors using Ficoll-Histopaque gradient density centrifugation. PBMCs were cultured in 96 well plates at a density of 2.5 x 10⁶ cells/ml in RPMI Glutamax containing 10% heat-inactivated FBS, 2mM non essential amino acids, 2mM sodium pyruvate, 50mM 2-mercaptoethanol, 100U/ml penicillin, 100ug/ml streptomycin and 50µg/ml gentamycin. Cells were treated with recombinant human Lrg1 200ng/ml and or human TGFβ1 5ng/ml and maintained at 37°C under 5% CO₂. After 4 days cells were harvested and washed once with PBS followed by acquisition of 20,000 cells per sample on a FACSCalibur Flow Cytometer. FACs data were analysed using FSC versus SSC using Flow Jo software.

Treatment of the PBMCs with TGFβ increased the population of CD14⁺/CD11b⁺ cells from 0.362% (control, Figure 7, top left panel) to 1.23% (Figure 7, top right panel). Treatment of the PBMC with Lrg1 also increased the population of CD14⁺/CD11b⁺ cells compared with the control PBMC sample (to 1.79%, Figure 7, bottom left panel). However, treatment of PBMCs with both TGFβ and Lrg1 increased the population of CD14⁺/CD11b⁺ cells to 4.74%. These data illustrate that Lrg1 is capable of modulating immune responses in a TGFβ-dependent manner, and further suggests that a tumour producing Lrg1 (as demonstrated in Example 1 above) would be capable of modulating a patient's immune response via this Lrg1 production. (Fig 8a)

We next investigated the effect that Lrg1 may have upon monocyte/macrophage phenotype in the context of tumour survival and expansion. Monocytes harvested from human peripheral blood mononuclear cells were treated for 48h with medium alone, TGFβ1, Lrg1 or TGFβ1 + Lrg1 and the expression of HLA-DR determined by flow cytometry. Treatment with 200ng/ml Lrg1 or a combination of 200ng/ml Lrg1 and 5ng/ml TGFß1 resulted in a significant reduction in the expression of HLA DR (Figure 8a). The percentage of HLA DRhi cells decreased and the percentage of HLA DR^{lo} cells increased on the CD14⁺ macrophages treated with Lrg-1 or Lrg-1+ TGFβ1 (Figure 8b). This is of relevance to cancer as tumour-associated macrophages (TAMs) that express low MHC class II are immunosuppressive and promote tumour angiogenesis. A small proportion of TAMs also express Tie2 and low levels of MHC class II, the so-called Tie2 expressing macrophages (TEMs), and deletion of TEMs has been found to greatly improve the efficacy of tumour therapy. We therefore investigated whether the HLA DR^{lo} CD14⁺ population induced in presence of Lrg-1 at 48h was both endoglin (CD105) and TIE2 positive. Flow cytometry revealed that the HLA DR^{lo} CD14⁺ population also expressed endoglin (Figure 8c) and TIE2 (Figure 8d). Our demonstration that Lrg1 induces a population of CD14⁺ macrophages that are HLADR^{lo}, ENG^{hi}, TIE2⁺ is consistent with the TAM/TEM pro-tumourogenic macrophage population.

The inventors have therefore not only demonstrated that Lrg1 is able to exert a direct stimulatory effect on tumour cells, particularly tumour cell proliferation, but also that some tumours are able to produce their own endogenous supply of Lrg1. Further, the inventors have demonstrated for the first time that tumours may be able to modulate the immune response via Lrg1 expression, making targeting of tumour environment immune cell function by antagonising Lrg1 expression a potential anti-cancer therapy.

### References

WO 96/38579
WO 2011/027129
US 2005/0064516
WO 2008/092214
US 2007/0184503
US 5,654,151
US 5,503,978
US 5,567,588
Haupt H, Baudner S; Hoppe Seylers Z (1977) Isolation and characterization of an unknown, leucine-rich 3.1-S-alpha2-glycoprotein from human serum Physiol Chem. Jun; 358(6): 639-46. (Title translated from original German)
Takahashi N, Takahashi Y, Putnam FW; (1985) Periodicity of leucine and tandem repetition of a 24-amino acid segment in the primary structure of leucine-rich alpha 2-glycoprotein of human serum Proc Natl Acad Sci USA. Apr;82(7):1906-10.
Lynch J, Fay J, Meehan M, Bryan K, Watters K, Murphy D, Stallings R (2012) MiRNA-335 suppresses neuroblastoma cell invasiveness by direct targeting of multiple genes from the non-canonical TGFβ signalling pathway Carcinogenesis Mar 1 (epub ahead of print)
Sun D, Kar S, Carr BI (1995) Differentially expressed genes in TGF-beta 1 sensitive and resistant human hepatoma cells Cancer Lett. Feb 10; 89(1):73-9.
Li X, Miyajima M, Jiang C, Arai H (2007) Expression of TGF-betas and TGF-beta type II receptor in cerebrospinal fluid of patients with idiopathic normal pressure hydrocephalus Neurosci Lett. Feb 14; 413(2):141-4. Epub 2006 Dec 27.
Meulmeester, E. and ten Dijke, P. (2011). The dynamic roles of TGF-β in cancer. J. Pathol. 223: 205-218.
Flavell, R.A., Sanjabi, S., Wrzesinski, S.H. and Licona-Limon, P. (2010). The polarisation of immune cells in the tumour environment by TGFβ. Nat. Rev. Immunol. 10:554-567.
Daly, A.C., Randall, R.A and Hill, C.S. (2008). Transforming growth factor beta-induced Smad1/5 phosphorylation in epithelial cells is mediated by novel receptor complexes and is essential for anchorage-independent growth. Mol. Cell Biol. 28:6889-6902
Zou, W. and Restifo, N.P. (2010). Th17 cells in tumour immunity and immunotherapy. Nat. Rev. Immunol. 10: 248-256.
Schmieder A, Schledzewski K, Michel J, Tuckermann JP, Tome L, Sticht C, Gkaniatsou C, Nicolay JP, Demory A, Faulhaber J, Kzhyshkowska J, Géraud C, Goerdt S. (2011). Synergistic activation by p38MAPK and glucocorticoid signaling mediates induction of M2-like tumor-associated macrophages expressing the novel CD20 homolog MS4A8A. Int J Cancer. 129: 122-132.
Welford AF, Biziato D, Coffelt SB, Nucera S, Fisher M, Pucci F, Di Serio C, Naldini L, De Palma M, Tozer GM, Lewis CE. (2011). TIE2-expressing macrophages limit the therapeutic efficacy of the vascular-disrupting agent combretastatin A4 phosphate in mice. J Clin Invest. 121: 1969-1973.

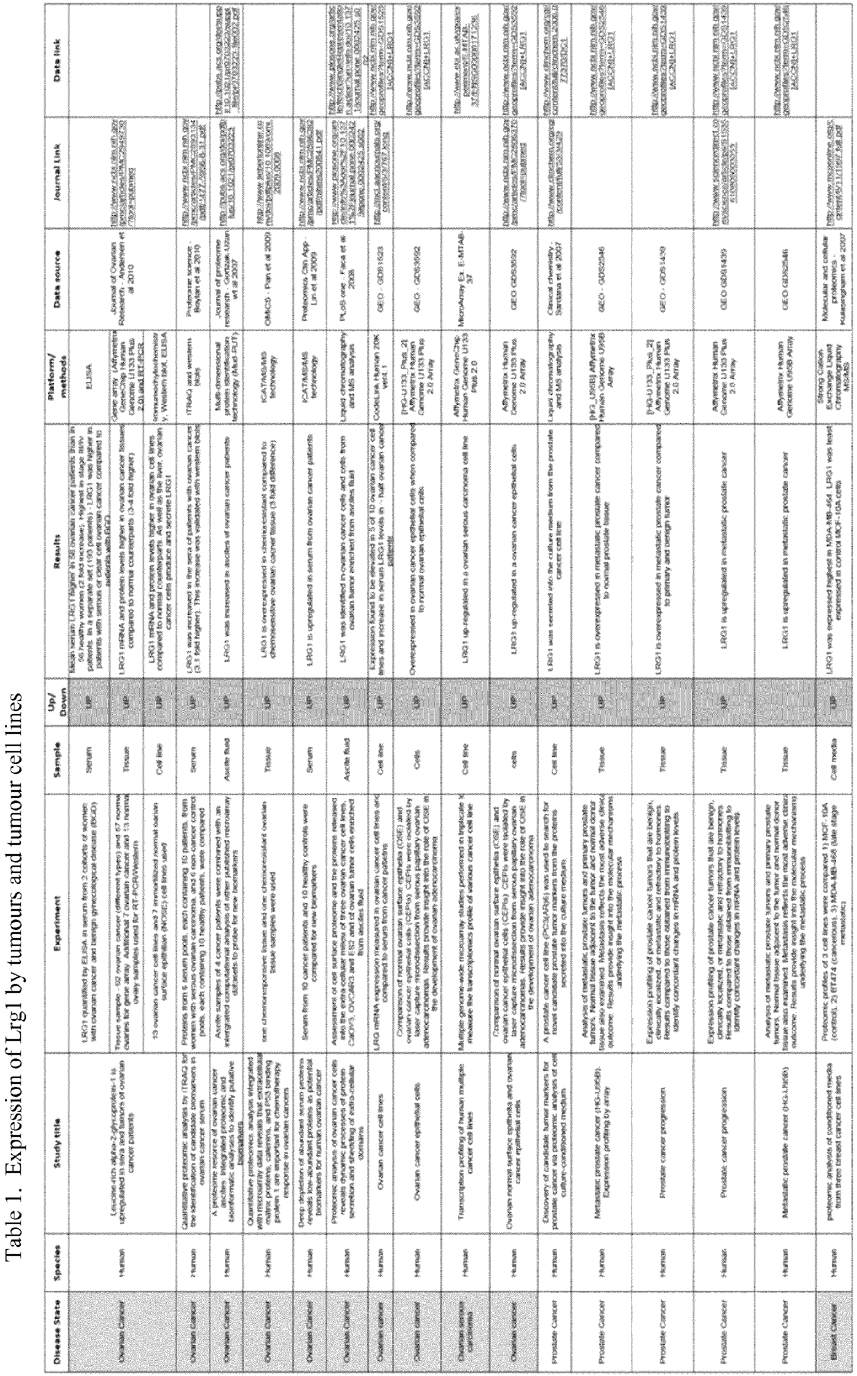

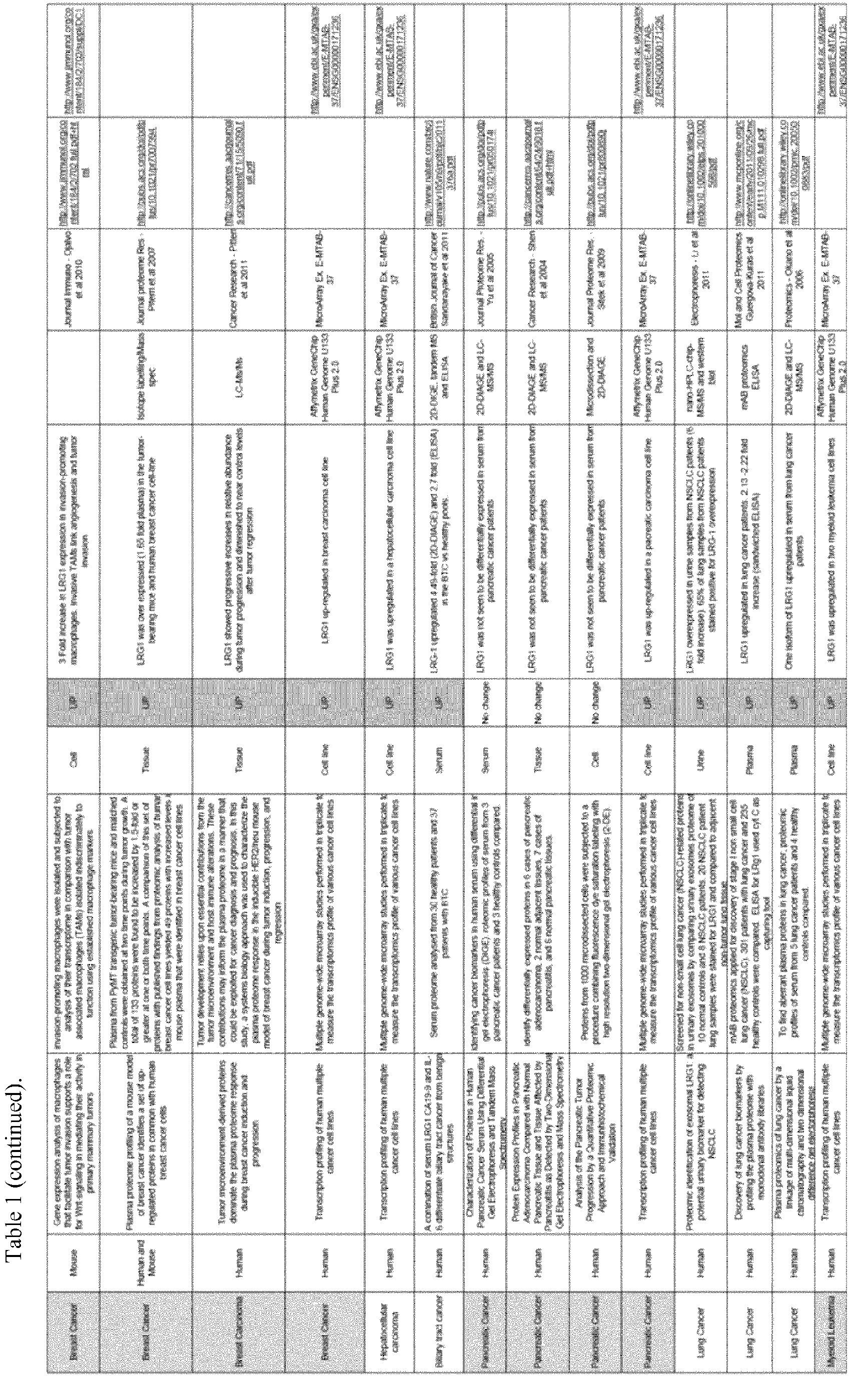

**Table 2: Expression of Lrg1 and endoglin in human normal and tumour epithelial cell lines.**

| **Cell line** | **Description** | **Secreted Lrg1** | **Endoglin** |
|---|---|---|---|
| MCF10A | Human mammary epithelial cell line | Y | Y |
| A549 | Human epithelial lung carcinoma | Y | Y |
| MDA MB 468 | Human mammary adenocarcinoma | N | N |
| LL2 | Murine Lewis lung carcinoma | Y | N/D |

### Appendix 1

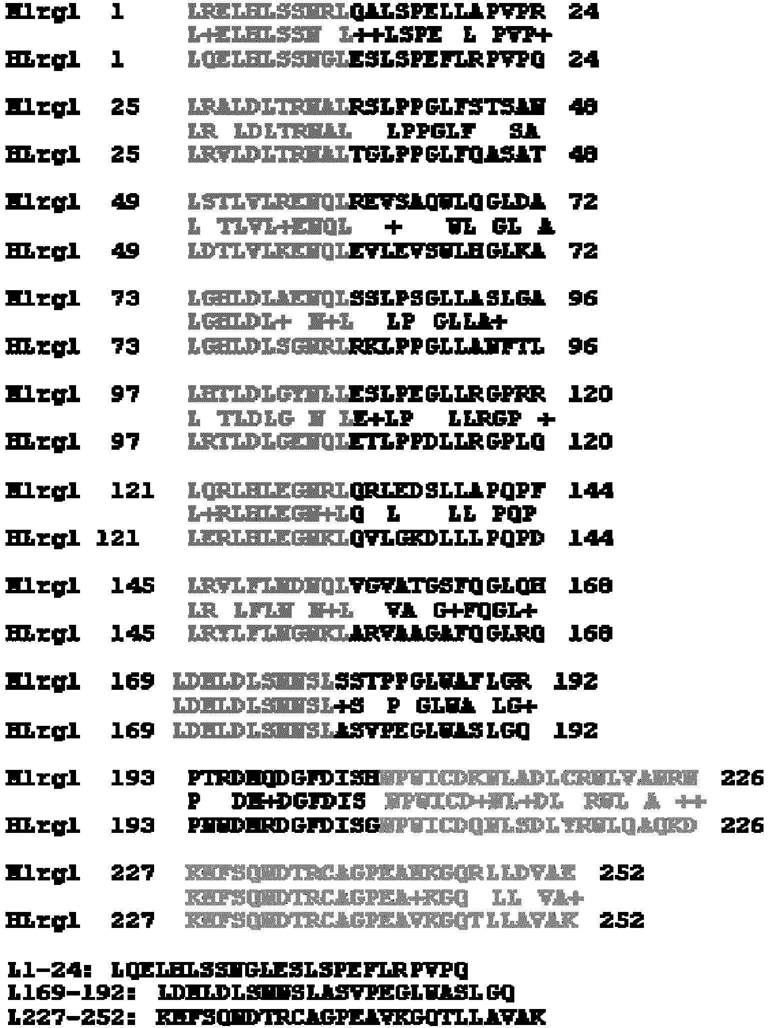

Partial sequence alignment of mouse and human Lrg1, arranged to illustrate the leucine-rich repeats (red), and the highly conserved C-terminal domains (green).

### Appendix 2

Human 1 MSSWSRQRPK SPGGIQPHVS RTLFLLLLLA ASAWGVTLSP M SW Q L LL G S
Mouse 1 MVSWQHQGSL QDLKTCLART LFLLALL--- ----GRVSSL
Human 41 KDCQVFRSDH GSSISCQPPA EIPGYLPADT VHLAVEFFNL K+C + +S GS++SC P E P LPADT VHL+VEF NL
Mouse 34 KECLILQSAE GSTVSCHGPT EFPSSLPADT VHLSVEFSNL
Human 81 THLPANLLQG ASKLQELHLS SNGLESLSPE FLRPVPQLRV T LPA LQG L+ELHLS SN L++LSPE L PVP+LR
Mouse 74 TQLPAAALQG CPGLRELHLS SNRLQALSPE LLAPVPRLRA
Human 121 LDLTRNALTG LPPGLFQASA TLDTLVLKEN QLEVLEVSWL LDLTRNAL LPPGLF SA L TLVL+EN QL + WL
Mouse 114 LDLTRNALRS LPPGLFSTSA NLSTLVLREN QLREVSAQWL
Human 161 HGLKALGHLD LSGNRLRKLP PGLLANFTLL RTLDLGENQL GL ALGHLD L+ N+L LP GLLA+ L TLDLG N L
Mouse 154 QGLDALGHLD LAENQLSSLP SGLLASLGAL HTLDLGYNLL
Human 201 ETLPPDLLRG PLQLERLHLE GNKLQVLGKD LLLPQPDLRY E+LP LLRGP +L+RLHLE GN+LQ L LL PQP LR
Mouse 194 ESLPEGLLRG PRRLQRLHLE GNRLQRLEDS LLAPQPFLRV
Human 241 LFLNGNKLAR VAAGAFQGLR QLDMLDLSNN SLASVPEGLW LFLN N+L VA G+FQGL+ LDMLDLSNN SL+S P GLW
Mouse 234 LFLNDNQLVG VATGSFQGLQ HLDMLDLSNN SLSSTPPGLW
Human 281 ASLGQPNWDM RDGFDISGNP WICDQNLSDL YRWLQAQKDK A LG+P DM +DGFDIS NP WICD NL+DL RWL A ++K
Mouse 274 AFLGRPTRDM QDGFDISHNP WICDKNLADL CRWLVANRNK
Human 321 MFSQNDTRCA GPEAVKGQTL LAVAKSQ MFSQNDTRCA GPEA+KGQ L L VA+
Mouse 314 MFSQNDTRCA GPEAMKGQRL LDVAELGSL

Leucine-rich α-2-glycoprotein 1 (Lrg1) exhibited the greatest fold change in the remodelled retinal vessels. Aligned amino acid sequence of human and mouse Lrg1. In red are the leucine rich repeat regions and in green is the human C-terminal domain region used as a blocking peptide.

### SEQUENCE INFORMATION

### Sequences of human Lrg1

***SEQ ID NO**: **1*** - *DNA Sequence of human Lrg1*
   [Sequence encoding protein of SEQ ID NO: 2 is **bold and underlined** within SEQ ID NO: 1 below]
*SEQ ID NO: 2 - Amino Acid Sequence of human Lrg1*
   [Sequences of SEQ ID NOS: 3-5 are **bold and underlined** within SEQ ID NO: 2 below]
***SEQ ID NOS: 3-5 - Amino Acid Sequences of Peptides within Lrg1***
***SEQ ID NO: 3** - Amino Acids 1 - 24 of human Lrg1 **from Appendix 2 (L94-117 from Appendix 3)***
   L1-24/L94-117: LQELHLSSNGLESLSPEFLRPVPQ
*SEQ ID NO: 4 - Amino Acids 169-192 of human Lrg1 **from Appendix 2 (L262-285** from Appendix 3)*
   L169-192/L262-285: LDMLDLSNNSLASVPEGLWASLGQ
*SEQ ID NO: **5** - Amino Acids 227-252 of human Lrg1 **from Appendix 2 (L320-345 from Appendix 3)***
   L227-252/L320-345: KMFSQNDTRCAGPEAVKGQTLLAVAK

## Claims

1. An antagonist of Leucine-rich alpha-2-glycoprotein 1 (Lrg1) for use in the treatment of a cancer that is not dependent on vasculoproliferation for growth or that is non-responsive to treatment with an anti-angiogenic or anti-vasculoproliferative agent, the antagonist comprising an antibody that binds to Lrg1, a double-stranded RNA that targets Lrg1 RNA, an anti-sense RNA that targets Lrg1 RNA, an aptamer that binds to Lrg1, or a peptide or peptidomimetic that competes with full-length Lrg1 and hence blocks Lrg1 function.

2. A Lrg1 antagonist for use according to claim 1, which acts on non-vascular cells.

3. A Lrg1 antagonist for use according to claim 1 or 2 wherein the antagonist down-regulates neoplastic cell proliferation.

4. A Lrg1 antagonist for use according to claim 3, wherein the Lrg1 antagonist has at least one additional effect on neoplastic cells selected from:
(a) down-regulation of neoplastic cell migration;
(b) down-regulation of cell-cell interactions between neoplastic cells;
(c) down-regulation of expression of neoplastic genes by neoplastic cells; and
(d) blocking the switch of TGFβ from an anti- to a pro-oncogenic factor for neoplastic cells.

5. A Lrg1 antagonist for use according to claim 1 or 2, wherein the antagonist:
(a) down-regulates neoplastic cell migration;
(b) down-regulates cell-cell interactions between neoplastic cells;
(c) down-regulates expression of neoplastic genes by neoplastic cells; or
(d) blocks the switch of TGFβ from an anti- to a pro-oncogenic factor for neoplastic cells.

6. An antagonist of Leucine-rich alpha-2-glycoprotein 1 (Lrg1) for use according to claim 1, wherein the treatment of cancer is by an effect on tumour environment immune cell function.

7. A Lrg1 antagonist for use according to claim 6, wherein:
(a) said antagonist decreases the percentage of CD14 positive CD11b positive cells within a peripheral blood mononuclear cell (PBMC) population compared to a control in which the antagonist is not administered; and/or
(b) said antagonist increases the percentage of RORγt positive CD4 T cells compared to a control in which the antagonist is not administered.

8. A Lrg1 antagonist for use according to any one of claims 1 to 7, wherein said antagonist blocks the interaction between:
(a) Lrg1 and TGFβ Receptor II (TGFβRII); and/or
(b) Lrg1 and TGFβ and/or
(c) Lrg1 and an activin receptor-like kinase (ALK) and/or
(d) Lrg1 and endoglin; and/or
(e) Lrg1 and betaglycan; and/or
(f) Lrg1 and a bone morphogenic protein (BMP); and/or
(g) Lrg1 and a bone morphogenic protein receptor (BMPR); and/or
(h) ALK and BMPR and/or
(i) Lrg1 and activin type II receptor (ACVRII); and/or
(j) endoglin and ALK; and/or
(k) ALK and BMPR; and/or
(l) ALK and TGFβRII, in TGFβ or BMP signalling,
optionally wherein said blocking by said antagonist:
(i) reduces the interaction between endoglin and Lrg1 and thereby modulates the interaction between the ALK and TGFβ Receptor II (TGFβRII); and/or
(ii) reduces the interaction between betaglycan and Lrg1, and thereby modulates the interaction between the ALK and TGFβRII; and/or
(iii) disrupts the formation of a BMP, BMPR and ALK complex, or the signalling by said complex; and/or
(iv) disrupts non-canonical TGFβ signalling; and/or
(v) disrupts the formation of a BMP, ACVRII and ALK complex, or the signalling by said complex.

9. A Lrg1 antagonist for use according to any one of the preceding claims, wherein:
(a) the antagonist double-stranded RNA is a short interfering RNA (siRNA) or microRNA (miRNA); or
(b) the antagonist peptide is a fragment of Lrg1, preferably which comprises one or more of sequences L1-24 (SEQ ID NO: 3), L169-192 (SEQ ID NO: 4), and L227-252 (SEQ ID NO: 5) or a part thereof, further preferably which comprises or consists of amino acids 227-252 of Lrg1.

10. An antagonist antibody for use according to any one of claims 1 to 8, which is a monoclonal antibody or a fragment of a monoclonal antibody.

11. An antagonist monoclonal antibody for use according to claim 10, which specifically recognises an epitope within the sequence of L1-24 (SEQ ID NO: 3), L169-192 (SEQ ID NO: 4) or L227-252 (SEQ ID NO: 5) of Lrg1, optionally which specifically recognises an epitope within L227-252 (SEQ ID NO: 5) of Lrg1.

12. A Lrg1 antagonist for use according to any one of the preceding claims, for use in combination with:
(a) another anti-cancer therapeutic, optionally
wherein the other anti-cancer therapeutic is selected from a cytotoxic agent, a chemotherapeutic agent, a growth inhibitory agent and an anti-cancer monoclonal antibody; and/or
(b) an anti-angiogenic compound, preferably wherein the antiangiogenic compound is an antagonist of vascular endothelial growth factor (VEGF), an angiopoietin antagonist, an antagonist of placental growth factor (PLGF), an antagonist of endoglin, a CD160 antagonist or an antagonist of activin receptor-like kinase 1 (ALK1), further preferably wherein said VEGF antagonist is an anti-VEGF antibody.

13. A Lrg1 antagonist for use according to any one of the preceding claims wherein:
(a) the Lrg1 antagonist is for use in the treatment of a cancer that is selected from myeloma, leukaemia, brain tumour, breast tumour, kidney tumour, colorectal tumour, lung tumour, prostate tumour, head and neck tumours, stomach tumour, pancreatic tumour, skin tumour, cervical tumour, bone tumour, ovarian tumour, testicular tumour and liver tumours; and/or
(b) the Lrg1 antagonist is for intravenous, intramuscular, intradermal, intraocular, intraperitoneal, subcutaneous, spinal, parenteral, topical, epidermal, sub-dural, intra-cranial ventricular or mucosal administration.

## Patentansprüche

1. Antagonist von Leucin-reichem Alpha-2-glycoprotein 1 (Lrg1) zur Verwendung in der Behandlung von einem Krebs, der für ein Wachstum nicht von Vasculoproliferation abhängig ist, oder der nicht auf eine Behandlung mit einem anti-angiogenen oder anti-vasculoproliferativen Mittel anspricht, der Antagonist umfassend einen Antikörper, der an Lrg1 bindet, eine doppelsträngige RNA, die Lrg1-RNA targetiert, eine Antisense-RNA, die Lrg1-RNA targetiert, ein Aptamer, das an Lrg1 bindet, oder ein Peptid oder Peptidomimetikum, das mit Lrg1 voller Länge konkurriert und somit Lrg1-Funktion blockt.

2. Lrg1-Antagonist zur Verwendung nach Anspruch 1, der auf nicht vaskuläre Zellen wirkt.

3. Lrg1-Antagonist zur Verwendung nach Anspruch 1 oder 2, wobei der Antagonist eine Proliferation neoplastischer Zellen abwärtsreguliert.

4. Lrg1-Antagonist zur Verwendung nach Anspruch 3, wobei der Lrg1-Antagonist mindestens eine zusätzliche Wirkung auf neoplastische Zellen hat, ausgewählt aus:
(a) Abwärtsregulierung einer Migration von neoplastischen Zellen;
(b) Abwärtsregulierung von Zell-Zell-Interaktionen zwischen neoplastischen Zellen;
(c) Abwärtsregulierung einer Expression von neoplastischen Genen durch neoplastische Zellen; und
(d) Blocken der Umwandlung von TGFß von einem anti- zu einem a pro-onkogenen Faktor für neoplastische Zellen.

5. Lrg1-Antagonist zur Verwendung nach Anspruch 1 oder 2, wobei der Antagonist:
(a) eine Migration von neoplastischen Zellen abwärtsreguliert;
(b) eine Zell-Zell-Interaktionen zwischen neoplastischen Zellen abwärtsreguliert;
(c) eine Expression von neoplastischen Genen durch neoplastische Zellen abwärtsreguliert; oder
(d) die Umwandlung von TGFß von einem anti- zu einem a pro-onkogenen Faktor für neoplastische Zellen blockt.

6. Antagonist von Leucin-reichem Alpha-2-glycoprotein 1 (Lrg1) zur Verwendung nach Anspruch 1, wobei die Behandlung von Krebs durch eine Wirkung auf Tumorumgebungs-Immunzellfunktion ist.

7. Lrg1-Antagonist zur Verwendung nach Anspruch 6, wobei:
(a) der Antagonist den Prozentsatz von CD14 positiven CD11b positiven Zellen in einer Population von mononukleären Zellen des peripheren Bluts (PBMC) im Vergleich zu einer Kontrolle, bei der der Antagonist nicht verabreicht wird, verringert; und/oder
(b) der Antagonist den Prozentsatz von RORγt positiven CD4 T-Zellen im Vergleich zu einer Kontrolle, bei der der Antagonist nicht verabreicht wird, verringert.

8. Lrg1-Antagonist zur Verwendung nach einem der Ansprüche 1 bis 7, wobei der Antagonist die Interaktion zwischen den folgenden blockt:
(a) Lrg1 und TGFß-Rezeptor II (TGFßRII); und/oder
(b) Lrg1 und TGFß; und/oder
(c) Lrg1 und einer Activinrezeptor-ähnlichen Kinase (ALK); und/oder
(d) Lrg1 und Endoglin; und/oder
(e) Lrg1 und Betaglycan; und/oder
(f) Lrg1 und einem knochenmorphogenen Protein (BMP); und/oder
(g) Lrg1 und einem Rezeptor für knochenmorphogenes Protein (BMPR); und/oder
(h) ALK und BMPR; und/oder
(i) Lrg1 und Rezeptor für Activin-Typ II (ACVRII); und/oder
(j) Endoglin und ALK; und/oder
(k) ALK und BMPR; und/oder
(l) ALK und TGFßRII,
in TGFß- oder BMP-Signalisierung, optional wobei das Blocken durch den Antagonisten:
(i) die Interaktion zwischen Endoglin und Lrg1 und reduziert und dadurch die Interaktion zwischen dem ALK- und TGFß-Receptor II (TGFßRII) moduliert; und/oder
(ii) die Interaktion zwischen Betaglycan und Lrg1 reduziert und dadurch die Interaktion zwischen dem ALK und TGFßRII moduliert; und/oder
(iii) die Bildung eines BMP-, BMPR- und ALK-Komplexes, oder die Signalisierung durch den Komplex unterbricht; und/oder
(iv) eine nicht kanonische TGFß-Signalisierung unterbricht; und/oder
(v) die Bildung eines BMP-, ACVRII- und ALK-Komplexes, oder die Signalisierung durch den Komplex unterbricht.

9. Lrg1-Antagonist zur Verwendung nach einem der vorherigen Ansprüche, wobei:
(a) die doppelsträngige Antagonist-RNA eine kurze interferierende RNA (siRNA), oder microRNA (miRNA) ist; oder
(b) das Antagonist-Peptid ein Fragment von Lrg1 ist, das vorzugsweise eine oder mehrere der Sequenzen L1-24 (SEQ ID NO: 3), L169-192 (SEQ ID NO: 4), und L227-252 (SEQ ID NO: 5) oder einen Teil davon umfasst, das ferner vorzugsweise die Aminosäuren 227-252 von Lrg1 umfasst oder daraus besteht.

10. Antagonist-Antikörper zur Verwendung nach einem der Ansprüche 1 bis 8, der ein monoklonaler Antikörper oder ein Fragment eines monoklonalen Antikörpers ist.

11. Monoklonaler Antagonist-Antikörper zur Verwendung nach Anspruch 10, der spezifisch ein Epitop innerhalb der Sequenz von L1-24 (SEQ ID NO: 3), L169-192 (SEQ ID NO: 4) oder L227-252 (SEQ ID NO: 5) von Lrg1 erkennt, optional der spezifisch ein Epitop innerhalb von L227-252 (SEQ ID NO: 5) von Lrg1 erkennt.

12. Lrg1-Antagonist zur Verwendung nach einem der vorherigen Ansprüche, zur Verwendung in Kombination mit:
(a) einem anderen Therapeutikum gegen Krebs, optional wobei das andere Therapeutikum gegen Krebs ausgewählt ist aus einem chemotherapeutischen Mittel, einem Wachstumshemmermittel und einem monoklonalen Antikörper gegen Krebs; und/oder
(b) einer anti-angiogenen Verbindung, vorzugsweise wobei der antiangiogene Verbindung ein Antagonist von vaskulärem endothelialem Wachstumsfaktor (VEGF), an Angiopoietin-Antagonist, ein Antagonist von plazentarem Wachstumsfaktor (PLGF), ein Antagonist von Endoglin, ein CD160-Antagonist oder ein Antagonist von Activinrezeptor-ähnlicher Kinase 1 (ALK1) ist, ferner vorzugsweise, wobei der VEGF-Antagonist ein Anti-VEGF-Antikörper ist.

13. Lrg1-Antagonist zur Verwendung nach einem der vorherigen Ansprüche, wobei:
(a) der Lrg1-Antagonist zur Verwendung in der Behandlung von einem Krebs ist, der ausgewählt ist aus Gehirntumor, Brusttumor, Nierentumor, kolorektalem Tumor, Lungentumor, Prostatatumor, Kopf- und Halstumor, Magentumor, Bauchspeicheldrüsentumor, Hauttumor, Gebärmutterhalstumor, Knochentumor, Eierstocktumor, Hodentumor und Lebertumoren; und/oder
(b) der Lrg1-Antagonist für eine intravenöse, intramuskuläre, intradermale, intraokulare, intraperitoneale, subkutane, spinale, parenterale, topische, epiderme, subdurale, intrakraniale ventrikuläre oder mukosale Verabreichung ist.

## Revendications

1. Antagoniste de la alpha-2-glycoprotéine 1 riche en leucine (Lrg1) destiné à une utilisation dans le traitement d'un cancer non dépendant de la prolifération vasculaire pour la croissance ou qui ne répond pas au traitement par un agent anti-angiogène ou anti-vasculoproliférateur, l'antagoniste comprenant un anticorps qui se lie au Lrg1, un ARN à double brin qui vise l'ARN de Lrg1, un ARN antisens qui vise l'ARN de Lrg1, un aptamère qui se lie au Lrg1 ou un peptide ou un peptidomimétique qui rivalise avec un Lrg1 pleine longueur et par conséquent bloque la fonction de Lrg1.

2. Antagoniste de Lrg1 destiné à une utilisation selon la revendication 1, qui agit sur des cellules non vasculaires.

3. Antagoniste de Lrg1 destiné à une utilisation selon la revendication 1 ou 2, dans lequel l'antagoniste régule négativement la prolifération des cellules néoplasiques.

4. Antagoniste de Lrg1 destiné à une utilisation selon la revendication 3, l'antagoniste de Lrg1 présentant au moins un effet supplémentaire sur les cellules néoplasiques choisies parmi :
(a) la régulation négative de la migration des cellules néoplasiques ;
(b) la régulation négative des interactions intercellulaires entre les cellules néoplasiques ;
(c) la régulation négative de l'expression des gènes néoplasiques par des cellules néoplasiques ; et
(d) l'empêchement de la transition de TGFβ d'un facteur anti-oncogène à un facteur pro-oncogène pour des cellules néoplasiques.

5. Antagoniste de Lrg1 destiné à une utilisation selon la revendication 1 ou 2, dans lequel l'antagoniste :
(a) régule négativement la migration des cellules néoplasiques ;
(b) régule négativement les interactions intercellulaires entre les cellules néoplasiques ;
(c) régule négativement l'expression des gènes néoplasiques par des cellules néoplasiques ; ou
(d) empêche la transition de TGFβ d'un facteur anti-oncogène à un facteur pro-oncogène pour des cellules néoplasiques.

6. Antagoniste de la alpha-2-glycoprotéine 1 riche en leucine (Lrg1) destiné à une utilisation selon la revendication 1, dans lequel le traitement du cancer se fait par un effet sur la fonction des cellules immunitaires de l'environnement tumoral.

7. Antagoniste de Lrg1 destiné à une utilisation selon la revendication 6, dans lequel :
(a) ledit antagoniste diminue le taux de cellules positives CD14 positives et CD11b positives au sein d'une population de cellules mononucléaires du sang périphérique (PBMC) en comparaison avec un contrôle dans lequel l'antagoniste n'est pas administré ; et/ou
(b) ledit antagoniste augmente le taux de cellules CD4 T positives RORγt en comparaison avec un témoin dans lequel l'antagoniste n'est pas administré.

8. Antagoniste de Lrg1 destiné à une utilisation selon l'une quelconque des revendications 1 à 7, dans lequel ledit antagoniste empêche les interactions entre :
(a) Lrg1 et un récepteur II de TGFβ (TGFβRII) ; et/ou
(b) Lrg1 et TGFβ ; et/ou
(c) Lrg1 et une kinase du type récepteur d'activine (ALK) ; et/ou
(d) Lrg1 et endogline ; et/ou
(e) Lrg1 et bétaglycane ; et/ou
(f) Lrg1 et une protéine morphogénique osseuse (BMP) ; et/ou
(g) Lrg1 et un récepteur de protéine morphogénique osseuse (BMPR) ; et/ou
(h) ALK et BMPR ; et/ou
(i) Lrg1 et un récepteur II du type activine (ACVRII) ; et/ou
(j) endogline et ALK ; et/ou
(k) ALK et BMPR ; et/ou
(l) ALK et TGFβRII,
dans la signalisation du TGFβ ou du BMP,
éventuellement dans lequel ledit blocage par ledit antagoniste :
(i) réduit l'interaction entre l'endogline et le Lrg1 et module ainsi l'interaction entre le récepteur ALK et le récepteur II de TGFβ (TGFβRII) ; et/ou
(ii) réduit l'interaction entre le bétaglycane et Lrg1, et module ainsi l'interaction entre l'ALK et TGFβRII ; et/ou
(iii) perturbe la formation d'un complexe BMP, BMPR et ALK, ou la signalisation par ledit complexe ; et/ou
(iv) perturbe la signalisation non canonique du TGFβ ; et/ou
(v) perturbe la formation d'un complexe BMP, ACVRII et ALK, ou la signalisation par ledit complexe.

9. Antagoniste de Lrg1 destiné à une utilisation selon l'une quelconque des revendications précédentes, dans lequel :
(a) l'ARN antagoniste à double brin est un petit ARN interférent (ARNsi) ou microARN (ARNmi) ; ou
(b) le peptide antagoniste est un fragment de Lrg1, de préférence comprenant au moins une des séquences L1-24 (SEQ ID N° : 3), L169-192 (SEQ ID N° : 4), et L227-252 (SEQ ID N° : 5) ou une partie de celles-ci, en outre de préférence comprenant ou étant constitué des acides aminés 227 à 252 de Lrg1.

10. Anticorps antagoniste destiné à une utilisation selon l'une quelconque des revendications 1 à 8, qui est un anticorps monoclonal ou un fragment d'un anticorps monoclonal.

11. Anticorps monoclonal antagoniste destiné à une utilisation selon la revendication 10, qui reconnaît spécifiquement un épitope à l'intérieur de la séquence de L1-24 (SEQ ID N° : 3), L169-192 (SEQ ID N° : 4) ou L227-252 (SEQ ID N° : 5) de Lrg1, éventuellement qui reconnaît spécifiquement un épitope à l'intérieur de L227-252 (SEQ ID N° : 5) de Lrg1.

12. Antagoniste de Lrg1 destiné à une utilisation selon l'une quelconque des revendications précédentes, en vue d'une utilisation combinée avec :
(a) un autre traitement anticancéreux, l'autre traitement anticancéreux étant éventuellement choisi parmi un agent cytotoxique, un agent chimiothérapeutique, un agent inhibiteur de croissance et un anticorps monoclonal anticancéreux ; et/ou
(b) un composé anti-angiogénique, ledit composé anti angiogénique étant de préférence un antagoniste du facteur de croissance endothélial vasculaire (VEGF), un antagoniste angiopoïétine, un antagoniste du facteur de croissance plancentaire (PLGF), un antagoniste d'endogline, un antagoniste de CD160 ou un antagoniste de la kinase 1 du type récepteur d'activine (ALK1), en outre de préférence ledit antagoniste de VEGF étant un anticorps anti-VEGF.

13. Antagoniste de Lrg1 destiné à une utilisation selon l'une quelconque des revendications précédentes, dans lequel :
(a) l'antagoniste de Lrg1 est destiné à une utilisation dans le traitement d'un cancer choisi parmi le myélome, la leucémie, la tumeur cérébrale, la tumeur mammaire, la tumeur rénale, la tumeur colorectale, la tumeur pulmonaire, la tumeur prostatique, la tumeur de la tête et du cou, la tumeur de l'estomac, la tumeur pancréatique, la tumeur cutanée, la tumeur cervicale, la tumeur osseuse, la tumeur ovarienne, la tumeur testiculaire et les tumeurs hépatiques ; et/ou
(b) l'antagoniste de Lrg1 est destiné à une administration intraveineuse, intramusculaire, intradermique, intraoculaire, intrapéritonéale, sous-cutanée, vertébrale, parentérale, locale, épidermique, sous-durale, ventriculaire intracrânienne ou mucosale.
